# EUROPEAN PATENT APPLICATION

(11) **EP 4 779 020 A1**
(43) Date of publication of application: **22.07.2026**
(21) Application number: 25382020.3
(22) Date of filing: 15.01.2025
(51) Int. Cl.: C12N 9/88, C12P 19/00

(54) **HYALURONIDASE OBTAINED FROM PURPUREOCILLIUM SP. AND ITS USE**

(71) Applicant: Consejo Superior De Investigaciones Científicas - CSIC, 28006 Madrid (ES); Universidad Autónoma de Madrid, 28049 Madrid (ES)
(72) Inventor: PLOU GASCA, Francisco José, MADRID (ES); MINGUET LOBATO, Marina, MADRID (ES); CERVANTES DOMÍNGUEZ, Fadia Victoria, MADRID (ES); FERNÁNDEZ LOBATO, María, MADRID (ES)
(74) Representative: Cueto, Sénida

(57) **Abstract**

The invention discloses a polypeptide with hyaluronidase activity from *Purpureocillium* sp, comprising the amino acid sequence in SEQ. ID. No. 1 or a variant thereof, a polynucleotide comprising said polypeptide, a recombinant vector, a host cell and a method to produce the polypeptide of SEQ. ID. No. 1 or a variant thereof and its use to produce a mixture of hyaluronan oligosaccharides.

## Description

### Technical Field

The present invention relates to a hyaluronidase from *Purpureocillium sp.*, applications thereof and a method for the production thereof.

### Background

Hyaluronic acid or hyaluronan is an unbranched anionic glycosaminoglycan (GAG) composed by tandem β-GIcA(1,3)-β-GIcNAc(1,4) disaccharide units. GAGs provide critical flexibility and elasticity to the extracellular matrix (ECM) in multicellular organisms, facilitating intricate cellular interaction networks. Beyond their structural roles, GAGs also regulate cellular functions, offering valuable potential in both biotechnological and medical applications.

The molecular weight of HA significantly influences its physical, chemical, and biological properties. High molecular weight (HMW-HA) is typically over 1,000 kDa and can be composed of up to 25,000 disaccharide units, medium molecular weight (MMW-HA) ranges from 250 to 1,000 kDa, and low molecular weight (LMW-HA) falls between 4 and 250 kDa. Hyaluronan oligosaccharides (HAOS) generally refer to HA fragments composed of up to 20 monomers (approximately 4 kDa maximum).

In this context, low molecular weight hyaluronic acids (LMW-HAs) and hyaluronic acid-derived oligosaccharides (HAOs) have attracted increasing interest due to their unique therapeutic potential. While LMW-HAs play an important role in the anti-inflammatory response and chronic wound healing, HAOs selectively kill tumour cells by disrupting the HA-receptor interaction. In addition, HAOs have been reported to enhance the sensitivity of cancer cells to drugs. The unique physicochemical properties and bioactivities of LMW-HAs and HAOS are highly chain length dependent. Unlike long-chain hyaluronic acid (HMW-HA), the smaller fragments penetrate the skin and promote cell regeneration, which has led to their use in cosmetic and dermatological products for skin care and prevention of skin ageing. In addition, their applications in regenerative medicine are being explored, especially in tissue engineering and joint injury repair.

The main problem with hyaluronidases is their low availability. At the commercial level, only those from bovine and ovine testis, and some bacterial ones such as *Streptomyces hyalurolyticus* are sold. They are marketed in very small quantities and at very high prices. This is why there is great interest in the development of fungal hyaluronidases, which could be produced on a large scale homologously or heterologously. Pang, B., Wang, H., Huang, H., Liao, L., Wang, Y., Wang, M., Du, G., & Kang, Z. (2022). Enzymatic production of low-molecular-weight hyaluronan and its oligosaccharides: A review and prospects. Journal of Agricultural and Food Chemistry, 70(44), 14129-14139*.*

The scientific articles EI-Safory, N. S., Lee, G. C., & Lee, C. K. (2011). Characterization of hyaluronate lyase from Streptococcus pyogenes bacteriophage H4489A. Carbohydrate Polymers, 84(3), 1182-1191. https://doi.org/10.1016/j.carbpol.2011.01.019; and Li, X., Li, F., Ma, J., Li, M., Lei, X., Tang, X., Wu, Q., Huang, Z., & Zhang, R. (2022). Biochemical and molecular characteristics of a novel hyaluronic acid lyase from Citrobacter freundii. Foods, 11(13), 1-13. https://doi.org/10.3390/foods11131989 disclose HylC hyaluronate lyase from *Citrobacter freundii* and the hyaluronate lyase from *S*. *pyogenes* bacteriophage H4489A both exhibit optimal activity at 37 °C and a pH of 5.5 a marked decrease in stability at higher temperatures. The VaHly8B hyaluronate lyase from *Vibrio alginolyticus* LWW-9 operates best at 50 °C and pH 7.0 but does not maintain activity as effectively beyond this point (Wang, X., Wei, Z., Wu, H., Li, Y., Han, F., & Yu, W. (2021). Characterization of a hyaluronic acid utilization locus and identification of two hyaluronate lyases in a marine bacterium Vibrio alginolyticus LWW-9. Frontiers in Microbiology, 12, 696096. https://doi.org/10.3389/fmicb.2021.696096), while the hyaluronate lyase from *S*. *hyalurolyticus* has optimal activity at 37 °C and pH 6.5 (Pang et al., 2022; and Stern, R., & Jedrzejas, M. J. (2006). Hyaluronidases: Their genomics, structures, and mechanisms of action. Chemical Reviews, 106(3), 818-839. https://doi.org/10.1021/cr050247k). Additionally, the BTH shows peak activity at 37 °C and pH 4.0, but then drops sharply and the LHyal hyaluronidase from leech *Hirudo nipponia*, which has been extensively exploited for the production of HAOS, operates optimally at 45 °C and pH 6.5 but after incubation at 60 °C its activity drops after 30 min (Jin, P., Kang, Z., Zhang, N., Du, G., & Chen, J. (2014). High-yield novel leech hyaluronidase to expedite the preparation of specific hyaluronan oligomers. Scientific Reports, 4, 4471. https://doi.org/10.1038/srep04471; and Pang et al., 2022). Furthermore, the patent document EP3010932 disclose a hyaluronidase from *Streptomyces koganeiensis* with maximum activity at physiological pH (around 7) and body temperature (about 37 °C);

None of these scientific articles and patents anticipate a hyaluronidase obtained from *Purpureocillium sp.* which has the combination of unique optimal conditions: particularly (1) ability to function at higher temperatures, (2) more acidic pH, (3) marked thermal stability and (4) producing a range of smaller oligomers from different sizes, compared to most bacterial HA lyases. These properties make it a promising and versatile enzyme for specialized applications requiring HA degradation under conditions that demand high-temperature resistance.

### Description

The present invention relates to a hyaluronidase from *Purpureocillium lilacinum.* Hyaluronidase activity has not been previously reported in *Purpureocillium sp.* As it will be shown later, the inventors have identified a novel hyaluronidase capable of efficiently transforming high molecular weight hyaluronic acid into different types of hyaluronan oligosaccharides.

For the purposes of this application, "hyaluronidase activity" refers to the enzymatic ability to degrade hyaluronic acid by cleaving the glycosidic bonds between N-acetylglucosamine and glucuronic acid, resulting in a reduction of hyaluronic acid polymer size. The activity can be quantified by any suitable assay, including but not limited to spectrophotometric methods, viscosity reduction, zymography, or other biochemical techniques that measure the degradation of hyaluronic acid or its derivatives.

Hyaluronidase activity includes not only the full catalytic activity observed in wild-type or optimized enzymes but also encompasses partial, reduced, or modified activity relative to a reference enzyme, as determined under comparable conditions.

A lyase-type protein of 44 kDa (PILya44; A0A179GQM7), hitherto unclassified, was identified and isolated from the transcriptome and secretome of *Purpureocillium sp.*, *a*nd its role in the degradation of hyaluronic acid was confirmed. PILya44 was successfully expressed in Pichia pastoris, and its production was scaled up to fermenter level, achieving a significant protein yield of 1.0 g/L. The enzyme demonstrated remarkable thermal stability, efficiently degrading high molecular weight hyaluronic acid at 50 °C and pH 4.0, while most bacterial hyaluronate lyases typically display optimal activity in the temperature range of 37-50 °C and a pH range of 6.0-8.0 (Stern, R., & Jedrzejas, M. J. (2006). Hyaluronidases: Their genomics, structures, and mechanisms of action. Chemical Reviews, 106(3), 818-839. https://doi.org/10.1021/cr050247k). This is particularly advantageous in industrial processes that require higher temperatures and more acidic pH

As it will be shown later PILya44 operates through an endolytic mechanism, cleaving β(1,4) glycosidic bonds to rapidly produce a variety of even-numbered unsaturated hyaluronan oligosaccharides, with dimers and tetramers as the primary end products. Furthermore, the enzyme is capable of cleaving β(1,3) glycosidic bonds, resulting in the generation of rare odd-numbered hyaluronan oligosaccharides, these odd-numbered hyaluronan oligosaccharides are difficult to generate, and exhibits the ability to degrade chondroitin sulphate, thereby underscoring its broader substrate specificity. These features make the enzyme of the invention very suitable for diverse industrial and biotechnological applications.

The first object of the invention relates to a polypeptide with hyaluronidase activity comprising:
the amino acid sequence in SEQ. ID. No. 1 or
a variant thereof, with an amino acid sequence having at least 80% at least 85%, at least 90% at least 95%, at least 96%, at least 97%, at least 98%, at least 99%, sequence identity over the whole length of SEQ ID No. 1.

In the present specification PILya44 and A0A179GQM7 are synonyms the amino acid sequence of A0A179GQM7 correspond to the polypeptide of SEQ. ID. No. 2. In the case of PILya44 it depends whether or not it comprises the native signaling peptide. This will be further discussed.

A0A179GQM7 is a Uniprot code of the Swiss-Prot/TrEMBL release 2024_06/2024_06 of November 27, 2024

In a particular embodiment the variant has an amino acid sequence with at least 80%, at least 81%, at least 82%, at least 83%, at least 84%; preferably at least 85%, at least 86%, at least 87%, at least 88%, at least 89%; more preferably at least 90%, at least 91%, at least 92%, at least 93%, at least 94%; even more preferably at least 95%, at least 96%, at least 97%, at least 98%, at least 99% sequence identity over the whole length of SEQ ID No. 2.

The difference between SEQ ID No. 1 and SEQ ID No. 2 is the first 21 amino acid residues, these are the signaling peptide, the function of the signaling peptide is to allow the cell to secrete the hyaluronidase to the extracellular space and it is excised during the process. A skilled person understands that endogenous signaling peptide can be replaced by another peptide which allows the cell to secrete the hyaluronidase. In the state of the art there are signaling peptides more efficient or adapted to specific cell hosts.

In a particular embodiment, the variants of SEQ ID No. 2 are at least 96%, at least 97%, at least 98%, at least 99%, sequence identity over the SEQ ID No. 1 from amino acid 22 to the end, this is excluding the native signal peptide. When comparing the percentage of identity of a polypeptide with SEQ ID No. 1 the signaling peptide of the polypeptide must be excluded from the analysis.

In a particular embodiment the polypeptide is extracellular.

In a particular embodiment the polypeptide of the invention SEQ ID No. 1 or variant thereof may comprise a signaling peptide, this signaling peptide can be found in any part of the polypeptide, preferably on the 5' end or 3' end of SEQ ID No. 1 or variant thereof; in a particular embodiment the signaling peptide is aMF disclosed in SEQ ID No. 3; SEQ ID No.4 discloses the hyaluronidase of the invention (SEQ ID No. 1) with the signaling peptide aMF

The polypeptide can be a recombinant polypeptide and in a particular embodiment it is an isolated polypeptide.

The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more (e.g., several) or all of the naturally occurring constituents with which it is associated in nature; (3) any substance modified by the hand of man relative to that substance found in nature; or (4) any substance modified by increasing the amount of the substance relative to other components with which it is naturally associated (e.g., multiple copies of a gene encoding the substance; use of a stronger promoter than the promoter naturally associated with the gene encoding the substance). An isolated substance may be present in a fermentation broth sample.

The hyaluronidase of the invention was identified in *Purpureocillium sp;* so it can be obtained from *Purpureocillium sp.;* preferably *Purpureocillium sp* is selected from the group consisting of *P*. *lilacinum, P. lavendulum* and *P*. *takamizusanensis* more preferably *Purpureocillium lilacinum* CECT 28398T.

*P. lilacinum*, formerly classified as *Paecilomyces lilacinus*, is a saprobic filamentous fungus commonly found in soil, decaying vegetation, and the rhizosphere of many crops. In another particular embodiment, *Purpureocillium lilacinum* is (known as *Paecilomyces lilacinus*) DSM 846 (CECT 20398 T).

As it will be shown later, the inventors surprisingly identified and isolated a 44 kDa lyase-type enzyme (PILya44) with hyaluronidase activity (UniProt ID: A0A179GQM7). Notably, and despite being present at the transcriptomic level, this enzyme had not been predicted by annotation tools. Analysis of the PILya44 amino acid sequence using InterPro identified it as a member of the "Chondroitin AC/Alginate Lyase" homologous superfamily (SSF48230) that contains an "Alginate Lyase" Pfam domain (PF05426). However, some annotation tools may omit sequences that do not show sufficient similarity to currently validated and classified proteins, further complicating the functional assignment process. Thus, experimental validation becomes essential for elucidating the actual functions of these proteins. This is, the predicted annotated functions of "Chondroitin AC/Alginate Lyase" an "Alginate Lyase" cannot predict the hyaluronidase function that this disclosure has identified. Moreover, later will be show that the hyaluronidase of the invention does not have Alginate Lyase activity (TABLE 3)

The term "variant" means a polypeptide having hyaluronidase activity comprising a substitution at one or more (e.g., several) positions i.e. a variant is also a polypeptide of the present invention. A substitution means replacement of the amino acid occupying a position with a different amino acid.

The variants have at least 20%, e.g., at least 40%, at least 50%, at least 60%, at least 70%, at least 80%, at least 90%, at least 95%, or at least 100% of the hyaluronidase activity of SEQ ID No. 1.

In an embodiment, the number of amino acid substitutions introduced into the polypeptide of SEQ ID No. 1 is 1-50, 1-40, 1-30, 1-20, 1-10 or 1-5, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 35, 36, 37, 38, 39, 40, 41, 42, 43, 44, 45, 46, 47, 48, 49 or 50.

The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described. Common substitutions are Ala/Ser, Val/lle, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/lle, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Another object of the invention relates to a polynucleotide comprising the nucleotide sequence shown in SEQ. ID. No. 5 encoding the bacterial hyaluronidase described above, or a variant thereof having at least 80% at least 85%, at least 90% at least 95%, at least 96%, at least 97%, at least 98%, at least 99%, sequence identity over the whole length of SEQ ID No. 5, the bacterial hyaluronidase maintaining hyaluronidase activity.

In a particular embodiment, the polynucleotide or the variant thereof is isolated.

In the present specification the polynucleotide that codifies for PILya44 /A0A179GQM7 is SEQ. ID. No. 6. NCBI Reference Sequence: XM_018324517.1; PLN 10-JUN-2024 consulted on January 12, 2025.

In a particular embodiment the variant has a polynucleotide with at least 80%, at least 81%, at least 82%, at least 83%, at least 84%; preferably at least 85%, at least 86%, at least 87%, at least 88%, at least 89%; more preferably at least 90%, at least 91%, at least 92%, at least 93%, at least 94%; even more preferably at least 95%, at least 96%, at least 97%, at least 98%, at least 99% sequence identity over the whole length of SEQ ID No. 5.

The difference between SEQ ID No. 5 and SEQ ID No. 6 is the first 63 nucleotide residues, these are the signaling peptide, the function of the signaling peptide is to allow the cell to secrete the hyaluronidase to the extracellular space. A skilled person understands that endogenous signaling peptide can be replaced by another peptide which allows the cell to secrete the hyaluronidase. in the state of the art there are signaling peptides more efficient or adapted to specific cell hosts.

In a particular embodiment, the variants of SEQ ID No. 6 are at least 96%, at least 97%, at least 98%, at least 99%, sequence identity over the SEQ ID No. 5 from polynucleotide 67 to the end, this is excluding the native signal peptide. When comparing the percentage of identity of a polynucleotide with SEQ ID No. 5 the signaling peptide of the polynucleotide must be excluded from the analysis.

In a particular embodiment the polynucleotide of the invention SEQ ID No. 5 or variant thereof may comprise a string of nucleotides codifying for a signaling peptide, this region can be found in any part of the polynucleotide, preferably on the 5' end or 3' end of SEQ ID No. 5 or variant thereof; in a particular embodiment the string of nucleotides is SEQ ID No. 7 that codifies for the signaling peptide aMF (SEQ ID No. 3). SEQ ID No. 8 discloses the polynucleotide of the invention with the polynucleotide region that codifies for the aMF signaling peptide at the 5' end.

The term "nucleic acid" is synonym to "polynucleotide" and as used in this description includes genes or gene fragments, as well as, in general, any DNA or RNA molecule, single or double stranded.

In another particular embodiment, the nucleotide sequence of SEQ ID NO: 4 could be codon-optimized for the expression in any host (a particular organism or cell, e.g., bacteria or yeast.) in, at least a 10% of the codons, preferably at least 20% of the codons, more preferably at least 30% of the codons, even more preferably at least 40% of the codons, even more preferably at least 50% of the codons, even more preferably at least 60% of the codons, even more preferably at least 70% of the codons, even more preferably at least 80% of the codons, and even more preferably at least 90% of the codons.

In a particular embodiment, the polynucleotide encoding the bacterial hyaluronidase of the invention can be optimized for a particular host. In a particular embodiment the host is P. *pastoris* and the optimized codon sequence is SEQ ID No. 9 or a variant thereof. A skilled person would know how to obtain an optimized polynucleotide sequence by using any free online tool such as http://genomes.urv.es/OPTIMIZER/

In another particular embodiment, the optimized codon sequence may comprise a polynucleotide codifying for a signalling peptide linked to any part of the sequence, preferably the 5' end or the 3' end of SEQ ID No. 9; said polynucleotide can be codon-optimized or not. The optimized codon sequence for *P*. *pastoris* with the polynucleotide sequence of the native signalling peptide of *Purpureocillium sp* is disclosed in SEQ ID No. 10; The optimized codon sequence for *P*. *pastoris* with the polynucleotide sequence of the signaling peptide aMF is disclosed in SEQ ID No. 11.

Another object of the invention relates to a genetically engineered recombinant vector comprising the polynucleotide described above, such as polynucleotide SEQ ID No. 5, a variant thereof or SEQ ID No. 9, SEQ ID No. 10 and/or SEQ ID No. 11.

In a particular embodiment, the vector is a plasmid, any plasmid that can be used to transform eukaryotic or prokaryotic host cells. A skilled person would know which suitable vectors can be used. For example, in *E. coli* pET expression vectors such as pET-21a, pET-28a, pET22b, pET28b, pGEX, and pBAD; in yeast, such as S. *cerevisiae,* for example, pYES2, and pRS; in *P. pastoris* for example, pPICZ, and pGAPZ; in mammalian cells, for example, pcDNA3.1, pIRES, pCMV-Tag; in insect cells, for example pFastBac, and pIB/V5-His.

In another particular embodiment, the is plasmid selected from the group consisting of: pGem-T, pIB4, pSTBlue and pPICZ, preferably a pGem-T plasmid or a plB4 vector.

Disclosed are also recombinant expression vectors comprising the polynucleotide disclosed above. The various nucleotide and control sequences may be joined together to produce a recombinant expression vector that may include one or more convenient restriction sites to allow for insertion or substitution of the polynucleotide of the invention at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, i.e., a vector that exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are *Bacillus licheniformis* or *Bacillus subtilis* dal genes, or markers that confer antibiotic resistance such as ampicillin, chloramphenicol, kanamycin, neomycin, spectinomycin or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to amdS (acetamidase), argB (ornithine carbamoyltransferase), bar (phosphinothricin acetyltransferase), hph (hygromycin phosphotransferase), niaD (nitrate reductase), pyrG (orotidine-5'-phosphate decarboxylase), sC (sulfate adenyltransferase), and trpC (anthranilate synthase), as well as equivalents thereof.

The choice of the promoter and terminator sequences and optionally signal or transit sequences, is determined, for example, on the basis of when, where, and how the hyaluronidases of the invention or the variant thereof is desired to be expressed. For instance, the expression may be constitutive or inducible. As it will be shown later in the examples, some recombinant vectors comprise the AOX1 promoter which is inducible with methanol. A skilled person would know that there are many examples of promoters in the field that can be used for a particular application.

Another object of the invention relates to a host cell comprising the vector as described above.

The host cell may be any cell useful in the recombinant production of a variant, e.g., a prokaryote or a eukaryote.

The term "host cell" means any cell type that is susceptible to transformation, transfection, transduction, or the like with a nucleic acid construct or expression vector comprising a polynucleotide of the present invention. The term "host cell" encompasses any progeny of a parent cell that is not identical to the parent cell due to mutations that occur during replication.

The prokaryotic host cell may be any Gram-positive or Gram-negative bacterium. Gram-positive bacteria include, but are not limited to *Bacillus*, *Clostridium*, *Enterococcus*, *Geobacillus*, *Lactobacillus, Lactococcus, Oceanobacillus*, *Staphylococcus, Streptococcus,* and *Streptomyces.* Gram-negative bacteria include, but are not limited to, *Campylobacter*, *E. coli, Flavobacterium, Fusobacterium, Helicobacter, Ilyobacter, Neisseria, Pseudomonas, Salmonella*, and *Ureaplasma.*

The bacterial host cell may be any *Bacillus* cell including, but not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells.

The bacterial host cell may also be any *Streptococcus* cell including, but not limited to, *Streptococcus equisimilis, Streptococcus pyogenes, Streptococcus uberis, and Streptococcus equi subsp. Zooepidemicus* cells.

The bacterial host cell may also be any *Streptomyces* cell, including, but not limited to, *Streptomyces achromogenes, Streptomyces avermitilis, Streptomyces coelicolor, Streptomyces griseus, and Streptomyces lividans* cells.

The host cell may also be a eukaryote, such as a mammalian, insect, plant, or fungal cell.

The host cell may be a fungal cell. "Fungi" as used herein includes the phyla *Ascomycota, Basidiomycota, Chytridiomycota,* and *Zygomycota* as well as the *Oomycota* and all mitosporic fungi.

The fungal host cell may be a yeast cell. "Yeast" as used herein includes ascosporogenous yeast (*Endomycetales*), basidiosporogenous yeast, and yeast belonging to the Fungi Imperfecti (Blastomycetes).

The yeast host cell may be a *Candida, Hansenula, Kluyveromyces, Pichia, Saccharomyces, Schizosaccharomyces,* or *Yarrowia* cell such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

The fungal host cell may be a filamentous fungal cell. "Filamentous fungi" include all filamentous forms of the subdivision Eumycota and Oomycota. The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as Saccharomyces cerevisiae is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Mucor circinelliodes, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

In a particular embodiment the host is P. *pastoris,* or *E. coli* more preferably strain *P. pastoris* GS115.

In order to ensure a better safety in the use of this bacterial enzyme in pharmaceutical applications, the isolated and characterized hyaluronidase from the original bacterial strain iwas produced in recombinant form using a non-pathogenic bacterial strain as a host cell defined as Generally Regarded as Safe (GRAS). The production of the bacterial recombinant from the "GRAS" micro-organism retains efficacy comparable to that of autologous hyaluronidase but with a higher amount of production per liter and with a substantially better profile of purity and safety, including the complete absence of the risk of viral transmission.

Another object of the invention relates to a method to produce the polypeptide with hyaluronidase SEQ ID No. 1 or a variant thereof from a host cell comprising the polynucleotide SEQ ID No 5. or a variant thereof described above comprising:
a. cultivating a host cell of the invention under conditions suitable for expression of the polypeptide; and
b. recovering a polypeptide of the invention.

The advantage of a heterologous expression in a host cell is that the hyaluronidase is the predominant protein in the extracellular fraction the host cell cultures, simplifying the downstream purification processes. In a particular embodiment the preferred host cell is P. *pastoris*, more preferably strain GS115; the vectors preferably expressed in said host cell are SEQ ID No. 9, SEQ ID No. 10 and/or SEQ ID No. 11.

The host cells are cultivated in a nutrient medium suitable for production of the variant using methods known in the art. For example, the cell may be cultivated by shake flask cultivation, or small-scale or large-scale fermentation (including continuous, batch, fed-batch, or solid state fermentations) in laboratory or industrial fermenters performed in a suitable medium and under conditions allowing the variant to be expressed and/or isolated. In a particular embodiment, the reaction can be a batch fermentation reaction or a fed-batch fermentation reaction preferably a fed-batch. A fed-batch reaction with an appropriate control of parameters give rise to a more than a 14-fold increase in protein production, and in less time. Moreover, the resulting protein has a higher enzyme activity.

In a particular embodiment the fermentation parameters are one or more of the following: Temperature from approximately 20°C to 40°C, more preferably from 25°C to 35°C, even more preferably 30 °C; Agitation from 300 rpm to 2000 rpm, preferably from 500 rpm to 1500 rpm, even more preferably approximately 1,000 rpm; Dissolved O₂ from approximately 10% (v/v) to 30% (v/v), more preferably from 15% (v/v) to 25% (v/v), even more preferably 20% (v/v); pH, between 3.5 and 8, preferably 4 and 7.5, more preferably 5 and 7, even more preferably approximately 6.0 for 60 to 150 h, preferably 138 h. Methanol can be added over 1 to 4 days, preferably 3 days at a rate of 2.7-4.6 mLVh/L, resulting in a final culture OD600 of about 150.

Another object of the invention relates to the use of SEQ ID No. 1 or a variant thereof to produce a mixture of hyaluronan oligosaccharides from HMW-HA, MMW-HA, LMW-HA or mixtures thereof. Another object of the invention relates to a method to produce a mixture of hyaluronan oligosaccharides using SEQ ID No. 1 or a variant thereof.

The method of the present invention allows to obtain high amounts of a high quality recombinant bacterial hyaluronidase for several biotechnological applications, such as pharmaceutical applications. The high production of recombinant hyaluronidase in non-pathogenic microorganisms has the same efficacy as the traditional hyaluronidase but with a significantly better safety and profile.

This method comprises the steps of (1) putting in contact the SEQ ID No. 1 or a variant thereof with HA in a suitable medium, such as water, the HA selected from the group consisting of HMW-HA, MMW-HA, and LMW-HA or mixtures thereof, and (2) let the reaction happen for a suitable amount of time, for example at least 5 min, or for example between 5 min and 200 h; or 10 min and 200 h; or 20 min and 200 h; or 30 min and 200 h; or 45 min and 200 h; or 1 h and 200 h, or between 2 h and 100h, preferably between 2 h and 90 h, even more preferably between 65 and 80 h; and, at an appropriate temperature, such as between 39 to 55°C, preferably between 40 °C and 55°C, preferably between 44°C and 54°C, more preferably between 45°C and 53°C, even more preferably at approximately, 46°C, 47°C, 48°C, 49^{a}C, 50°C, 51°C, or 52°C; and at an appropriate pH, such as between 3.7 and 6, preferably 3.8 and 5.5, more preferably 3.9 and 5, even more preferably at approximately 4, 4.1, 4.2,4.3,4.4, 4.5, 4.6,4.7,4.8, or 4.9.

In a preferred embodiment, the temperature is between 45°C and 53°C and the pH between 3.9 and 5.

The ranges of temperature and pH disclosed in the preceding paragraphs cover the optimal activity of the enzyme of the invention. Its stability at elevated temperatures positions the recombinant bacterial hyaluronidase of the invention as a superior alternative to commonly used hyaluronidases, such as the bovine testicular hyaluronidase BTH and the leech hyaluronidase LHyal, for HAOS preparation (Pang et al., 2022).

As indicated previously the bacterial hyaluronidase of the invention primarily exhibits an endolytic mode of action. This results in the rapid production of a wide range of HAOS fragments. PILya44 ability to cleave both β(1,3) and β(1,4) glycosidic bonds adds to its versatility, generating a unique range of unsaturated oligomers, including rare, odd-numbered species

The amount of time conditions the different HAOS yield, as it will be shown later: The 3-hour and 72-hour reactions yielded different HAOS profiles, predominantly forming even-numbered unsaturated oligomers in substantial amounts.

The mixtures of hyaluronan oligosaccharides produced by the hyaluronidase of the invention exhibit significant antioxidant properties, which can be used for a range of biotechnological and industrial applications. As it will be shown later, while HMW HA showed minimal antioxidant activity, the HAOS mixtures, especially the 72-hour reaction product (HAOS-72h), exhibited significantly antioxidant potential

### List of sequences

**SEQ ID No. 1:** A0A179GQM7; NCBI Reference Sequence: XP_018177696.1 without the first 21 amino acid residues of the signaling peptide. 
APQRENAPTRTVEGDTAKRAVFKHPGIFVDSDRLKLMASKVAAAEQPWADAYSALKTHKYA
TRTKPTPYPTVECGPYSTPNVGCEDERADAMAAYANALMWAVTKDQAKADRAIEFMNAWA
KTVKSHTNSNAPLQGAWAAADWARAGEIIRYTNAGWSSADITTFENMLRNVYLPLVKPGSQ
NPNNWELMFNEAAISISVFLNDKATYDSTMTRFLRSAAQYFYLKSDGPRPVKPAGMTDEKQ
DWWWNGQIARGLEDGIAMEICRDLTHTGYGIASVSHVIETSKIQGRDLYNEEVGTRLRYALE
FQSKYDPKGGAVAAPSWLCDGTLKLHLEDVTEPGWNAMHHKYSMPWSGNFTLKHRPAGA
NTLFVGWETLTHAE
**SEQ ID No. 2:** A0A179GQM7; NCBI Reference Sequence: XP_018177696.1 
MKLLTTLPALAAMMHLAVVHGAPQRENAPTRTVEGDTAKRAVFKHPGIFVDSDRLKLMASK
VAAAEQPWADAYSALKTHKYATRTKPTPYPTVECGPYSTPNVGCEDERADAMAAYANALM
WAVTKDQAKADRAIEFMNAWAKTVKSHTNSNAPLQGAWAAADWARAGEIIRYTNAGWSSA
DITTFENMLRNVYLPLVKPGSQNPNNWELMFNEAAISISVFLNDKATYDSTMTRFLRSAAQY
FYLKSDGPRPVKPAGMTDEKQDWWWNGQIARGLEDGIAMEICRDLTHTGYGIASVSHVIET
 
SKIQGRDLYNEEVGTRLRYALEFQSKYDPKGGAVAAPSWLCDGTLKLHLEDVTEPGWNAM
HHKYSMPWSGNFTLKHRPAGANTLFVGWETLTHAE
**SEQ ID No. 3:** signaling peptide of *S*. *cerevisiae* aMF 
MRFPSIFTAVLFAASSALAAPVNTTTEDETAQIPAEAVIGYSDLEGDFDVAVLPFSNSTNNGL
LFINTTIASIAAKEEGVSLEKREAEA
**SEQ ID No. 4:** A0A179GQM7 with the first 21 amino acid residues of the signaling peptide replaced by αMF. 
MRFPSIFTAVLFAASSALAAPVNTTTEDETAQIPAEAVIGYSDLEGDFDVAVLPFSNSTNNGL
LFINTTIASIAAKEEGVSLEKREAEAAPQRENAPTRTVEGDTAKRAVFKHPGIFVDSDRLKLM
ASKVAAAEQPWADAYSALKTHKYATRTKPTPYPTVECGPYSTPNVGCEDERADAMAAYAN
ALMWAVTKDQAKADRAIEFMNAWAKTVKSHTNSNAPLQGAWAAADWARAGEIIRYTNAGW
SSADITTFENMLRNVYLPLVKPGSQNPNNWELMFNEAAISISVFLNDKATYDSTMTRFLRSA
AQYFYLKSDGPRPVKPAGMTDEKQDWWWNGQIARGLEDGIAMEICRDLTHTGYGIASVSH
VIETSKIQGRDLYNEEVGTRLRYALEFQSKYDPKGGAVAAPSWLCDGTLKLHLEDVTEPGW
NAMHHKYSMPWSGNFTLKHRPAGANTLFVGWETLTHAE
**SEQ ID No. 5:** *Purpureocillium lilacinum* uncharacterized protein (PLICBS_008008), partial mRNA; NCBI Reference Sequence: XM_018324517.1 without the region codifying for the native signaling peptide 
GCACCACAGCGGGAGAACGCCCCAACAAGGACTGTTGAAGGCGATACTGCCAAGCGC
GCAGTCTTCAAACACCCCGGCATTTTTGTCGATTCAGATCGCCTCAAGCTCATGGCGTC
CAAGGTGGCCGCCGCGGAGCAGCCGTGGGCCGATGCCTACAGCGCGCTCAAGACTCA
CAAGTACGCCACGCGGACCAAGCCGACGCCGTACCCGACGGTCGAGTGCGGCCCGTA
CTCGACCCCTAACGTCGGATGCGAGGACGAGAGGGCCGACGCCATGGCCGCGTACGC
CAACGCCCTCATGTGGGCCGTCACCAAGGACCAGGCCAAGGCGGACCGGGCCATCGA
ATTCATGAACGCCTGGGCCAAGACGGTCAAGTCGCACACCAACTCTAATGCGCCTCTG
CAGGGCGCCTGGGCAGCCGCAGACTGGGCGCGCGCGGGCGAGATCATCCGCTATAC
GAACGCCGGATGGAGCAGTGCCGACATCACCACCTTTGAGAACATGTTGCGGAATGTC
TATCTCCCTCTGGTCAAGCCAGGGTCCCAGAATCCCAACAACTGGGAGCTGATGTTCAA
CGAGGCAGCTATTAGCATATCCGTCTTCCTCAATGACAAAGCAACGTACGACTCCACCA
TGACGCGCTTCCTCAGGAGCGCAGCGCAGTATTTTTATCTCAAGAGCGACGGCCCGCG
ACCTGTCAAGCCCGCAGGCATGACGGACGAGAAGCAGGACTGGTGGTGGAATGGCCA
AATCGCACGGGGGCTCGAGGACGGCATCGCCATGGAGATCTGCCGAGACCTCACCCA
CACGGGCTACGGCATCGCGTCTGTCTCGCACGTCATTGAGACGTCCAAGATCCAGGGC
CGGGACCTGTACAACGAGGAGGTCGGCACTCGGCTGCGGTACGCGCTCGAGTTCCAG
TCCAAGTACGATCCCAAGGGCGGAGCCGTTGCAGCGCCGTCGTGGCTCTGCGACGGG
ACGCTGAAGCTGCACCTTGAGGACGTCACGGAGCCGGGCTGGAATGCCATGCACCACA
 
AGTACAGCATGCCGTGGTCTGGCAACTTCACCTTGAAGCACAGGCCTGCCGGCGCCAA
CACCTTGTTTGTTGGTTGGGAGACGCTGACCCATGCTGAGTGA
**SEQ ID No. 6:** *Purpureocillium lilacinum* uncharacterized protein (PLICBS_008008), partial mRNA; NCBI Reference Sequence: XM_018324517.1 
ATGAAGCTCCTCACAACCCTACCGGCCCTTGCGGCCATGATGCACCTGGCAGTGGTCC
ACGGCGCACCACAGCGGGAGAACGCCCCAACAAGGACTGTTGAAGGCGATACTGCCA
AGCGCGCAGTCTTCAAACACCCCGGCATTTTTGTCGATTCAGATCGCCTCAAGCTCATG
GCGTCCAAGGTGGCCGCCGCGGAGCAGCCGTGGGCCGATGCCTACAGCGCGCTCAAG
ACTCACAAGTACGCCACGCGGACCAAGCCGACGCCGTACCCGACGGTCGAGTGCGGC
CCGTACTCGACCCCTAACGTCGGATGCGAGGACGAGAGGGCCGACGCCATGGCCGCG
TACGCCAACGCCCTCATGTGGGCCGTCACCAAGGACCAGGCCAAGGCGGACCGGGCC
ATCGAATTCATGAACGCCTGGGCCAAGACGGTCAAGTCGCACACCAACTCTAATGCGC
CTCTGCAGGGCGCCTGGGCAGCCGCAGACTGGGCGCGCGCGGGCGAGATCATCCGC
TATACGAACGCCGGATGGAGCAGTGCCGACATCACCACCTTTGAGAACATGTTGCGGA
ATGTCTATCTCCCTCTGGTCAAGCCAGGGTCCCAGAATCCCAACAACTGGGAGCTGATG
TTCAACGAGGCAGCTATTAGCATATCCGTCTTCCTCAATGACAAAGCAACGTACGACTC
CACCATGACGCGCTTCCTCAGGAGCGCAGCGCAGTATTTTTATCTCAAGAGCGACGGC
CCGCGACCTGTCAAGCCCGCAGGCATGACGGACGAGAAGCAGGACTGGTGGTGGAAT
GGCCAAATCGCACGGGGGCTCGAGGACGGCATCGCCATGGAGATCTGCCGAGACCTC
ACCCACACGGGCTACGGCATCGCGTCTGTCTCGCACGTCATTGAGACGTCCAAGATCC
AGGGCCGGGACCTGTACAACGAGGAGGTCGGCACTCGGCTGCGGTACGCGCTCGAGT
TCCAGTCCAAGTACGATCCCAAGGGCGGAGCCGTTGCAGCGCCGTCGTGGCTCTGCG
ACGGGACGCTGAAGCTGCACCTTGAGGACGTCACGGAGCCGGGCTGGAATGCCATGC
ACCACAAGTACAGCATGCCGTGGTCTGGCAACTTCACCTTGAAGCACAGGCCTGCCGG
CGCCAACACCTTGTTTGTTGGTTGGGAGACGCTGACCCATGCTGAGTGA
**SEQ ID No. 7:** polynucleotide sequence for factor alpha of *Saccharomyces cerevisie*: 
ATGAGATTTCCTTCAATTTTTACTGCTGTTTTATTCGCAGCATCCTCCGCATTAGCTGCTC
CAGTCAACACTACAACAGAAGATGAAACGGCACAAATTCCGGCTGAAGCTGTCATCGGT
TACTCAGATTTAGAAGGGGATTTCGATGTTGCTGTTTTGCCATTTTCCAACAGCACAAAT
AACGGGTTATTGTTTATAAATACTACTATTGCCAGCATTGCTGCTAAAGAAGAAGGGGTA
TCTCTCGAGAAAAGAGAGGCTGAAGCT
**SEQ ID No. 8:** *Purpureocillium lilacinum* XM_018324517.1 with the region codifying for the native signaling peptide replaced by polynucleotide region for factor alpha of *Saccharomyces cerevisie* 
ATGAGATTTCCTTCAATTTTTACTGCTGTTTTATTCGCAGCATCCTCCGCATTAGCTGCTC
CAGTCAACACTACAACAGAAGATGAAACGGCACAAATTCCGGCTGAAGCTGTCATCGGT
 
TACTCAGATTTAGAAGGGGATTTCGATGTTGCTGTTTTGCCATTTTCCAACAGCACAAAT
AACGGGTTATTGTTTATAAATACTACTATTGCCAGCATTGCTGCTAAAGAAGAAGGGGTA
TCTCTCGAGAAAAGAGAGGCTGAAGCTGCACCACAGCGGGAGAACGCCCCAACAAGGA
CTGTTGAAGGCGATACTGCCAAGCGCGCAGTCTTCAAACACCCCGGCATTTTTGTCGAT
TCAGATCGCCTCAAGCTCATGGCGTCCAAGGTGGCCGCCGCGGAGCAGCCGTGGGCC
GATGCCTACAGCGCGCTCAAGACTCACAAGTACGCCACGCGGACCAAGCCGACGCCGT
ACCCGACGGTCGAGTGCGGCCCGTACTCGACCCCTAACGTCGGATGCGAGGACGAGA
GGGCCGACGCCATGGCCGCGTACGCCAACGCCCTCATGTGGGCCGTCACCAAGGACC
AGGCCAAGGCGGACCGGGCCATCGAATTCATGAACGCCTGGGCCAAGACGGTCAAGT
CGCACACCAACTCTAATGCGCCTCTGCAGGGCGCCTGGGCAGCCGCAGACTGGGCGC
GCGCGGGCGAGATCATCCGCTATACGAACGCCGGATGGAGCAGTGCCGACATCACCA
CCTTTGAGAACATGTTGCGGAATGTCTATCTCCCTCTGGTCAAGCCAGGGTCCCAGAAT
CCCAACAACTGGGAGCTGATGTTCAACGAGGCAGCTATTAGCATATCCGTCTTCCTCAA
TGACAAAGCAACGTACGACTCCACCATGACGCGCTTCCTCAGGAGCGCAGCGCAGTAT
TTTTATCTCAAGAGCGACGGCCCGCGACCTGTCAAGCCCGCAGGCATGACGGACGAGA
AGCAGGACTGGTGGTGGAATGGCCAAATCGCACGGGGGCTCGAGGACGGCATCGCCA
TGGAGATCTGCCGAGACCTCACCCACACGGGCTACGGCATCGCGTCTGTCTCGCACGT
CATTGAGACGTCCAAGATCCAGGGCCGGGACCTGTACAACGAGGAGGTCGGCACTCG
GCTGCGGTACGCGCTCGAGTTCCAGTCCAAGTACGATCCCAAGGGCGGAGCCGTTGCA
GCGCCGTCGTGGCTCTGCGACGGGACGCTGAAGCTGCACCTTGAGGACGTCACGGAG
CCGGGCTGGAATGCCATGCACCACAAGTACAGCATGCCGTGGTCTGGCAACTTCACCT
TGAAGCACAGGCCTGCCGGCGCCAACACCTTGTTTGTTGGTTGGGAGACGCTGACCCA
TGCTGAGTGA
**SEQ ID No: 9:** Optimized codon sequence for *P*. *pastoris* as host cell without signalling peptide. 
GCTCCACAAAGAGAGAACGCTCCAACTAGAACTGTTGAGGGTGACACTGCTAAGAGAG
CCGTTTTTAAGCACCCAGGTATCTTCGTTGACTCCGACAGATTGAAGTTGATGGCTTCCA
AGGTTGCTGCTGCTGAACAACCATGGGCTGATGCTTACTCCGCTCTTAAGACTCACAAG
TACGCCACCAGAACTAAGCCAACTCCATATCCAACCGTTGAGTGTGGTCCATACTCCAC
TCCAAACGTTGGTTGTGAAGATGAACGTGCTGATGCTATGGCTGCTTACGCTAACGCTT
TGATGTGGGCTGTTACTAAGGACCAAGCTAAGGCTGACAGAGCCATCGAATTCATGAAC
GCTTGGGCTAAGACTGTCAAGTCCCACACTAACTCTAACGCCCCATTGCAAGGTGCTTG
GGCTGCTGCAGATTGGGCTAGAGCTGGTGAGATTATCAGATACACTAACGCTGGTTGGT
CCTCCGCTGACATTACTACTTTCGAGAACATGCTGAGGAACGTCTACTTGCCATTGGTC
AAGCCAGGTTCTCAGAACCCAAACAACTGGGAGTTGATGTTCAACGAGGCTGCTATTTC
CATCTCCGTGTTCTTGAACGACAAGGCTACTTACGACTCCACCATGACCAGATTCTTGA
GATCCGCTGCTCAGTACTTCTACTTGAAGTCTGACGGTCCAAGACCAGTTAAGCCAGCT
 
GGTATGACTGACGAAAAGCAAGATTGGTGGTGGAACGGTCAGATCGCTAGAGGTTTGG
AAGATGGTATCGCCATGGAAATCTGCAGAGACTTGACTCACACTGGTTACGGTATCGCT
TCTGTTTCCCACGTTATCGAGACTTCCAAGATCCAGGGTAGAGACTTGTACAACGAAGA
GGTCGGTACTAGATTGAGATACGCCTTGGAGTTCCAGTCCAAGTACGATCCAAAAGGTG
GTGCTGTTGCTGCTCCATCTTGGTTGTGTGATGGTACTTTGAAGCTGCACTTGGAGGAC
GTTACTGAACCAGGTTGGAACGCTATGCACCACAAGTACTCTATGCCATGGTCCGGTAA
CTTCACCTTGAAGCACAGACCTGCTGGTGCTAACACTTTGTTCGTTGGTTGGGAAACTTT
GACCCACGCTGAGTAA
**SEQ ID No. 10:** Optimized codon sequence for *P*. *pastoris* as host cell with polynucleotide sequence of the native signalling peptide of *Purpureocillium lilacinum.* 
ATGAAGTTGCTGACTACCTTGCCAGCTTTGGCTGCTATGATGCACTTGGCTGTTGTTCAT
GGTGCTCCACAAAGAGAGAACGCTCCAACTAGAACTGTTGAGGGTGACACTGCTAAGA
GAGCCGTTTTTAAGCACCCAGGTATCTTCGTTGACTCCGACAGATTGAAGTTGATGGCT
TCCAAGGTTGCTGCTGCTGAACAACCATGGGCTGATGCTTACTCCGCTCTTAAGACTCA
CAAGTACGCCACCAGAACTAAGCCAACTCCATATCCAACCGTTGAGTGTGGTCCATACT
CCACTCCAAACGTTGGTTGTGAAGATGAACGTGCTGATGCTATGGCTGCTTACGCTAAC
GCTTTGATGTGGGCTGTTACTAAGGACCAAGCTAAGGCTGACAGAGCCATCGAATTCAT
GAACGCTTGGGCTAAGACTGTCAAGTCCCACACTAACTCTAACGCCCCATTGCAAGGTG
CTTGGGCTGCTGCAGATTGGGCTAGAGCTGGTGAGATTATCAGATACACTAACGCTGGT
TGGTCCTCCGCTGACATTACTACTTTCGAGAACATGCTGAGGAACGTCTACTTGCCATT
GGTCAAGCCAGGTTCTCAGAACCCAAACAACTGGGAGTTGATGTTCAACGAGGCTGCTA
TTTCCATCTCCGTGTTCTTGAACGACAAGGCTACTTACGACTCCACCATGACCAGATTCT
TGAGATCCGCTGCTCAGTACTTCTACTTGAAGTCTGACGGTCCAAGACCAGTTAAGCCA
GCTGGTATGACTGACGAAAAGCAAGATTGGTGGTGGAACGGTCAGATCGCTAGAGGTT
TGGAAGATGGTATCGCCATGGAAATCTGCAGAGACTTGACTCACACTGGTTACGGTATC
GCTTCTGTTTCCCACGTTATCGAGACTTCCAAGATCCAGGGTAGAGACTTGTACAACGA
AGAGGTCGGTACTAGATTGAGATACGCCTTGGAGTTCCAGTCCAAGTACGATCCAAAAG
GTGGTGCTGTTGCTGCTCCATCTTGGTTGTGTGATGGTACTTTGAAGCTGCACTTGGAG
GACGTTACTGAACCAGGTTGGAACGCTATGCACCACAAGTACTCTATGCCATGGTCCGG
TAACTTCACCTTGAAGCACAGACCTGCTGGTGCTAACACTTTGTTCGTTGGTTGGGAAA
CTTTGACCCACGCTGAGTAA
**SEQ ID No. 11:** Optimized codon sequence for *P*. *pastoris* as host cell with the polynucleotide sequence of the factor alpha signalling peptide of *S*. *cerevisiae.*

ATGAGATTTCCTTCAATTTTTACTGCTGTTTTATTCGCAGCATCCTCCGCATTAGCTGCTC
CAGTCAACACTACAACAGAAGATGAAACGGCACAAATTCCGGCTGAAGCTGTCATCGGT
TACTCAGATTTAGAAGGGGATTTCGATGTTGCTGTTTTGCCATTTTCCAACAGCACAAAT
AACGGGTTATTGTTTATAAATACTACTATTGCCAGCATTGCTGCTAAAGAAGAAGGGGTA

TCTCTCGAGAAAAGAGAGGCTGAAGCTGCTCCACAAAGAGAGAACGCTCCAACTAGAA
CTGTTGAGGGTGACACTGCTAAGAGAGCCGTTTTTAAGCACCCAGGTATCTTCGTTGAC
TCCGACAGATTGAAGTTGATGGCTTCCAAGGTTGCTGCTGCTGAACAACCATGGGCTGA
TGCTTACTCCGCTCTTAAGACTCACAAGTACGCCACCAGAACTAAGCCAACTCCATATC
CAACCGTTGAGTGTGGTCCATACTCCACTCCAAACGTTGGTTGTGAAGATGAACGTGCT
GATGCTATGGCTGCTTACGCTAACGCTTTGATGTGGGCTGTTACTAAGGACCAAGCTAA
GGCTGACAGAGCCATCGAATTCATGAACGCTTGGGCTAAGACTGTCAAGTCCCACACTA
ACTCTAACGCCCCATTGCAAGGTGCTTGGGCTGCTGCAGATTGGGCTAGAGCTGGTGA
GATTATCAGATACACTAACGCTGGTTGGTCCTCCGCTGACATTACTACTTTCGAGAACAT
GCTGAGGAACGTCTACTTGCCATTGGTCAAGCCAGGTTCTCAGAACCCAAACAACTGGG
AGTTGATGTTCAACGAGGCTGCTATTTCCATCTCCGTGTTCTTGAACGACAAGGCTACTT
ACGACTCCACCATGACCAGATTCTTGAGATCCGCTGCTCAGTACTTCTACTTGAAGTCT
GACGGTCCAAGACCAGTTAAGCCAGCTGGTATGACTGACGAAAAGCAAGATTGGTGGT
GGAACGGTCAGATCGCTAGAGGTTTGGAAGATGGTATCGCCATGGAAATCTGCAGAGA
CTTGACTCACACTGGTTACGGTATCGCTTCTGTTTCCCACGTTATCGAGACTTCCAAGAT
CCAGGGTAGAGACTTGTACAACGAAGAGGTCGGTACTAGATTGAGATACGCCTTGGAG
TTCCAGTCCAAGTACGATCCAAAAGGTGGTGCTGTTGCTGCTCCATCTTGGTTGTGTGA
TGGTACTTTGAAGCTGCACTTGGAGGACGTTACTGAACCAGGTTGGAACGCTATGCACC
ACAAGTACTCTATGCCATGGTCCGGTAACTTCACCTTGAAGCACAGACCTGCTGGTGCT
AACACTTTGTTCGTTGGTTGGGAAACTTTGACCCACGCTGAGTAA

### Brief description of the drawings

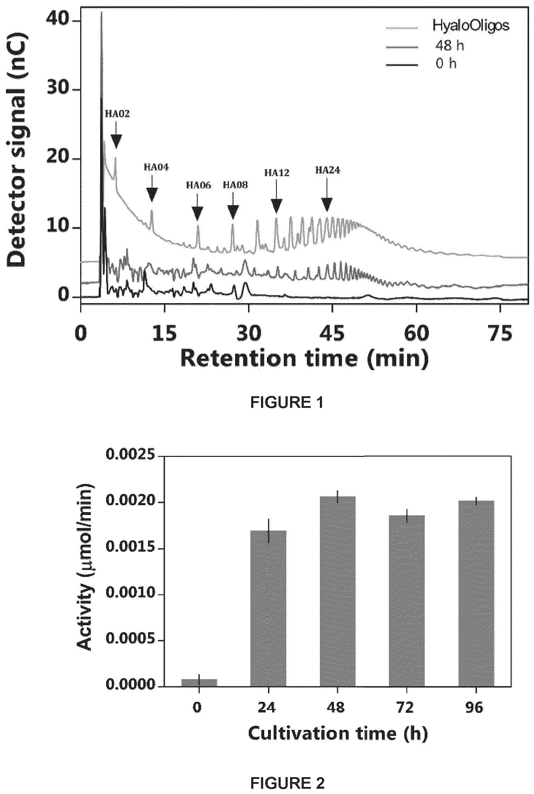

**FIGURE 1****. Representative HPAEC-PAD chromatographic profiles showing HAOS formation during *Purpureocillium sp.* cultivation.** Chromatograms of filtered extracellular culture media are shown for 0 h and 48 h of cultivation. HyaloOligos, a commercial low molecular weight saturated even-numbered hyaluronan oligosaccharide mixture with an average MW below 10 kDa, was prepared at 0.5 mg/mL, diluted, and injected for comparison. HA02 corresponds to the β-GIcA(1,3)-β-GIcNAc(1,4) HA dimeric unit, illustrating the nomenclature used for hyaluronan oligomers of varying degrees of polymerization.
**FIGURE 2****. Hyaluronidase activity during *Purpureocillium sp.* cultivation.** Time-course of hyaluronidase activity during the cultivation of *Purpureocillium sp.* from 0 to 96 hours. Reactions were performed for 1 hour at 30 °C and 900 rpm, using 0.1 M sodium acetate buffer (pH 6.0) and 0.25 g/L HMW HA.

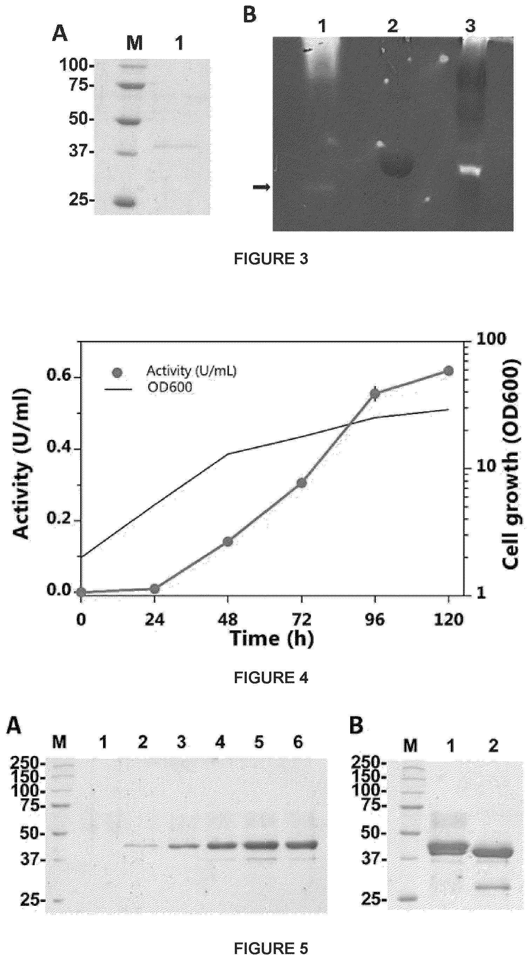

**FIGURE 3****. Analysis of the SEC-purified protein fraction. (A)** SDS-PAGE (12 %) analysis of the purified active protein fraction (lane 1). Molecular mass standards are indicated on the left in kDa, with lane M showing the marker. **(B)** Zymogram analysis using a 10 % polyacrylamide gel with 0.05 % hyaluronic acid (HA) as the substrate. Lane 1: purified protein fraction; lane 2: bovine serum albumin (66 kDa); lane 3: native bovine hyaluronidase (55 kDa). The arrow indicates the activity spot from SEC-purified protein fraction.
**FIGURE 4****. Hyaluronidase activity profile of PILya44 in *P. pastoris* cultures.** The graph presents cell growth (OD600, solid line) and extracellular hyaluronidase activity (blue circles) of P. *pastoris* cultures expressing PILya44 following methanol induction. The activity of cultures containing the empty plB4 vector served as a negative control (empty circles). Each data represents the mean of three independent experiments, and standard errors are indicated.
**FIGURE 5****. SDS-PAGE and glycosylation analysis of PILya44 expression in *P. pastoris* cultures. (A)** SDS-PAGE analysis of culture filtrates (15 µL) at 0, 24, 48, 72, 96, and 120 hours of methanol induction (lanes 1-6, respectively). **(B)** Glycosylation analysis of PILya44 (120-hour culture filtrate) before (lane 1) and after (lane 2) EndoH (29 kDa) treatment under denaturing conditions. Molecular mass markers (lane M) are shown on the left of each panel in kDa.

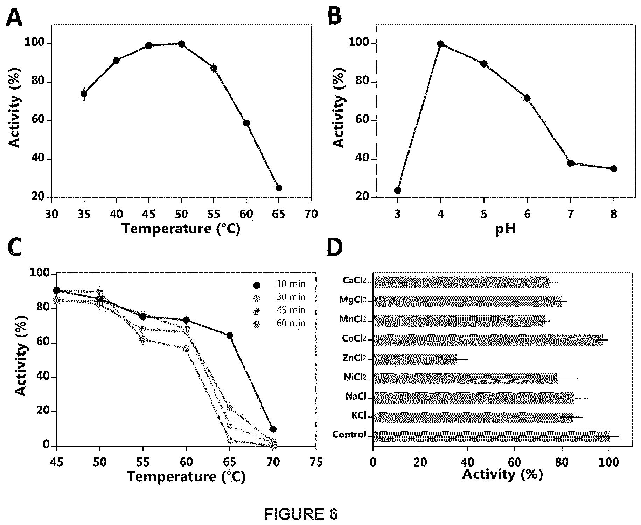

**FIGURE 6****. Influence of pH, temperature, and metal ions on the activity of PILya44. (A)** Optimal temperature for high molecular weight (HMW) hyaluronic acid (HA) degradation. **(B)** Optimal pH for HMW HA degradation. **(C)** Thermal stability of PILya44 at specified temperatures and incubation times. **(D)** Impact of metal ions on PILya44 activity. Percentages are calculated relative to the maximum activity observed in **(A)** and **(B),** the activity of proteins not exposed to the indicated temperatures in **(C),** and the activity measured in the absence of metal ions in **(D).** Data represent the average of three independent assays, with standard errors shown.

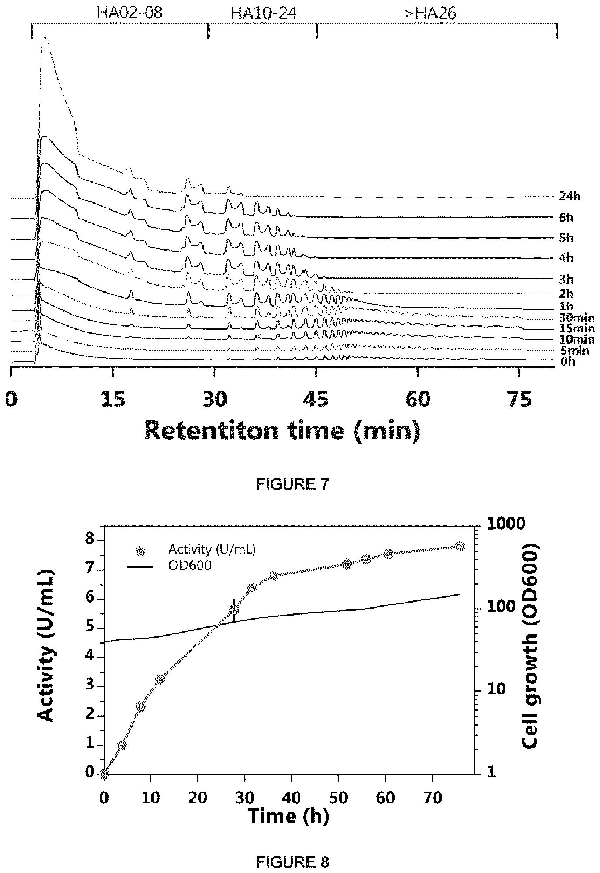

**FIGURE 7****. HPAEC-PAD chromatographic profiles showing the time-course degradation of HMW HA by the recombinant PILya44 enzyme.** Reactions were performed with 2.5 g/L of HMW HA over a 24-hour period at 50 °C in distilled water. The profiles illustrate the breakdown of HMW HA into various HAOS at different time points (0-24 h), as indicated on the right. Chromatographic profiles in blue (5 min, 30 min, 2 h, and 24 h) were further analysed via MALDI-TOF MS, with the identified HAOS detailed in TABLE 6 and TABLE 7. The DP of the formed HAOS is indicated at the top of the chromatograms.
**FIGURE 8****. Hyaluronidase activity profile of PILya44 in fed-batch fermentation.** The figure illustrates the hyaluronidase activity of PILya44 during fed-batch fermentation. The graph shows the correlation between cell growth (measured as OD600, represented by a solid line) and extracellular hyaluronidase activity (blue circles) in *P*. *pastoris* cultures expressing PILya44 following methanol induction. Each data point represents the mean of three independent experiments, and standard errors are indicated.

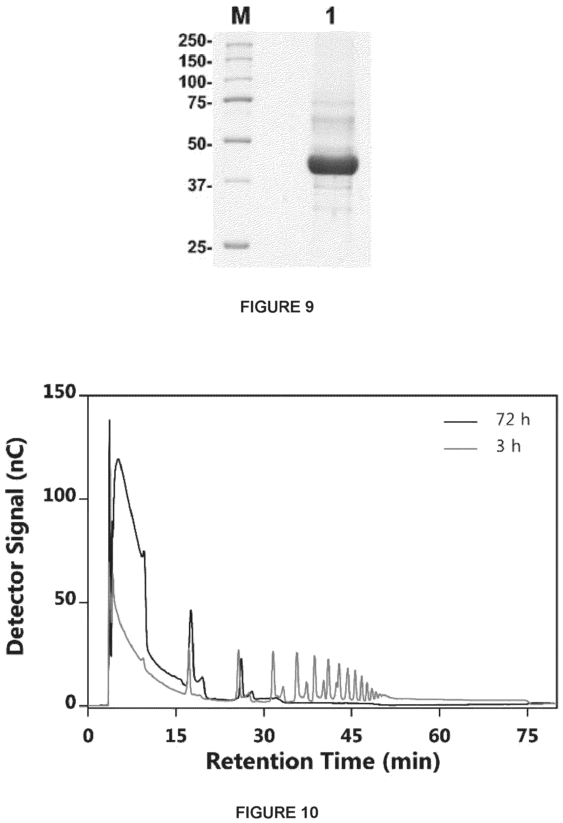

**FIGURE 9****. Analysis of the heterologous expression of PILya44 protein in *P. pastoris* using fed-batch fermentation.** A sample of culture filtrate (5 µL) was taken from yeasts cultivated in fed-batch conditions and induced with methanol for 76 hours (lane 1). The numbers on the left of the panel indicate molecular mass standards (lane M) in kDa.
**FIGURE 10****. HPAEC-PAD chromatographic profiles illustrating the HAOS generated during the degradation of HMW HA in scaled-up reactions by the action of the PILya44 enzyme.** Reactions were performed with 2.5 g/L of HMW HA over a 3-hour period (blue) and 72-hour period (black) at 50 °C in distilled water. Chromatographic profiles were further analysed via MALDI-TOF MS, with the identified HAOS detailed in TABLE 8 and TABLE 9.

### Examples

### Materials and Methods

### Reagents, materials, and buffer solutions

All solutions used in the present disclosure were prepared with either distilled or double-distilled water (Milli-Q Plus system, Millipore). Unless otherwise specified, molecular biology kits and commercial enzymes were used according to the instructions provided by the manufacturer.

### Biological material

### Bacteria

*Escherichia coli* DH5α (F-, ψ80dlacZΔM15, Δ(lacZYA-argF)U169, recA1, endA1, hsdR17 (rK-, mK+), phoA, supE44, λ-, thi-1, gyrA96, relA1) was supplied by the CBMSO (Centro de Biologia Molecular Severo Ochoa) and used as host for DNA manipulations.

### Yeasts and filamentous fungi

*Komagataellla phaffi* GS115 (*his4*-, Mut+), commonly known as *Pichia pastoris* GS115, was used as host for expression of proteins (Invitrogen). *Purpureocillium sp.* was from laboratory collection. *Purpureocillium lilacinum* (known as *Paecilomyces lilacinus*) DSM 846 (CECT 20398 T) and HRL-1 (CECT 20704) were from CECT. *Purpureocillium takamizusanensis* (CBS 132047) (known as *Isaria takamizusanensis*) and *Purpureocillium lavendulum* (CBS 128677) were from the Westerdijk Fungal Biodiversity Institute. *Purpureocillium* species were evaluated as potential hyaluronidase producers.

### Culture and maintenance of microorganisms

The composition of all media is based on a final volume of one liter of distilled water. Growth was monitored spectrophotometrically by measuring the optical density at 600 nm (OD600), using the specific sterilized medium as the reference. For solid media, 20 g of agar was added prior to sterilization.

### Bacteria

Luria-Bertani (LB) medium was used for the growth and maintenance of *E. coli* cells. The bacteria were cultured at 37 °C, with orbital shaking at 250 rpm in liquid media. Bacterial transformants were selected on LB media containing 100 mg/L ampicillin.

### Yeasts

*P. pastoris* cultures at 28-30 °C, both with orbital shaking at 200 rpm in liquid media. The yeasts were maintained on Yeast Extract Peptone Dextrose (YEPD) solid medium at 4 °C. *P. pastoris* transformants were selected and stored (4 °C) on Minimal Dextrose (MD) solid medium (Yeast Nitrogen Base without Amino Acids (YNB); Biotin and D-glucose). The mixture was gently agitated, aliquoted into cryovial tubes, and stored at -70 °C. Buffered minimal media with glycerol (BMG) or methanol (BMM) were used for cultivating transformants and analysing protein expression (YNB, biotin and glycerol/methanol). To prevent precipitation, 100 mL/L of 1 M potassium phosphate buffer (pH 6.0) was added to the BMG and BMM media after sterilization, along with methanol, as described below in section *Recombinant protein production in flask cultures.*

The production of the hyaluronidase of the invention was scaled up in a fed-batch fermenter using the PTM1 (CuSO₄·5H₂O 6.0 g; Nal 0.08 g; MnSO₄·H₂O 3.0 g; Na₂MoO₄·2H₂O 0.2 g; H₃BO₃ 0.02 g; CoCl₂ 0.5 g; ZnCl₂ 20.0 g; FeSO₄·7H₂O 65.0 g; H₂SO₄ 5 mL; Biotin 0.2 g) and Fermentation Basal Salts (FBS) (H₃PO₄ 26.7 mL; CaSO₄ 0.9 g; K₂SO₄ 18.2 g; MgSO₄·7H₂O 14.9 g; KOH 4.1 g; Glycerol 40 g; PTM1 medium 12 mL) media. PTM1 medium was sterilized by filtration and stored at room temperature until use. Following sterilization and cooling, the glycerol (sterilized separately) was combined with the FBS and PTM1 media, and the pH was adjusted to 6.0.

### Filamentous fungi

Cultures of *Purpureocillium sp., P. lilacinum* and *P*. *lavendulum* were incubated at 26-28 °C, while *P*. *takamizusanensis* was incubated at 20-22 °C, with orbital shaking at 200 rpm in liquid cultures. These fungal microorganisms were cultivated and maintained at 4 °C in liquid or solid PDA medium.

Hyaluronidase expression was induced by growing the fungi in Hyaluronate Induction (HAI) medium (High Molecular Weight Hyaluronate 0.2-2 g; Peptone 1.0 g; NaNO₃ 1.0 g; MgSO₄·7H₂O 0.5 g Casamino Acids 1.0 g). After sterilization, 100 mL/L of 1 M potassium phosphate buffer (pH 6.0) was added.

### Transformation of microorganisms

### Bacterial transformation

Transformation of *E. coli* cells were conducted using the conventional heat shock method. For plasmid DNA transformations, 5-10 µL of DNA at 50-500 ng/µL was added to competent cells. In the case of amplicon-based transformations, an equivalent volume (5-10 µL) was utilized, with DNA concentrations ranging from 10 to 50 ng/µL for each amplicon. These parameters were meticulously optimized to maximize transformation efficiency.

### Yeasts transformation

To prepare *P*. *pastoris* competent cells, 5 mL of a pre-inoculum of this organism was cultivated overnight into YEPD medium. Fresh YEPD medium (0.2 mL in a 1 L flask) was inoculated using 0.05-0.1 mL of the overnight culture. Cells were cultivated to an OD600 of 1.3-1.5, then harvested by centrifugation (2,000 rpm, 4 °C, 6 min), washed twice with 200 mL ice-cold sterilized water, and then with 10 mL ice-cold 1M sorbitol. Finally, the cells were resuspended in 1 mL sorbitol and kept on ice. For transformation, 80 µL of competent cells were mixed with 10-30 µg of linearized plasmid DNA in ice-cold electroporation cuvettes (0.2 cm, Bio-Rad). Electroporation was performed using the Gene Pulser II and Pulse Controller system (Bio-Rad) with settings of 1.5 kV, 25 µF and 200 Ω. Immediately after pulsing, 1 mL of ice-cold 1 M sorbitol was added to the cuvette and the mixture was transferred to a sterile 12 mL tube and incubated at 30 °C without shaking for 1-2 hours. The cells were then spread on MD plates and incubated for 2-3 days until colonies appeared.

### Handling and analysis of nucleic acids

### Plasmid DNA

Routine extraction of plasmidic DNA from *E. coli* cells was performed using the NZYMiniprep kit. For the transformation of *P*. *pastoris* competent cells, plasmidic DNA was extracted using the alkaline lysis method known in the art.

### Yeast genomic DNA

Yeast genomic DNA (gDNA) was extracted following the protocol described by Latorre-Garcia, L., Del Castillo-Agudo, L., & Polaina, J. (2007). Taxonomical classification of yeasts isolated from kefirbased on the sequence of their ribosomal RNA genes. World Journal of Microbiology and Biotechnology, 23(6), 785-791. https://doi.org/10.1007/s11274-006-9298-y with minor modifications. Yeasts were cultured overnight (12-16 h) in 5 mL of YEPD, then resuspended in a final concentration of 3 % SDS (w/v). The mixture was heated at 55 °C for 15 minutes and subsequently placed on ice for 10 min after adding potassium acetate to a final concentration of 0.5 M. Cellular debris was removed by centrifugation at 4 °C and 13,000 rpm. DNA was precipitated by adding an equal volume of cooled isopropanol, recovered by centrifugation as before, and treated with 20 mg/L RNase A for 30 min.

### DNA fragment synthesis

When necessary, synthetic genes were assembled at the GeneArt Gene Synthesis Facility of Thermo Fisher Scientific and optimized for *P*. *pastoris* codon usage by implementing the GeneOptimizer algorithm of Invitrogen to enhance the downstream protein expression.

### Electrophoresis, quantification and purification of nucleic acids

Polymerase chain reaction (PCR) products were analysed on 0.7-0.8 % agarose gels containing 1.5 µL GreenSafe. The gels were prepared and run in TAE buffer. Samples were prepared using 1X Gel Loading Dye Purple. Electrophoresis was conducted at 100 V for 30 min, with Ladder III serving as molecular weight marker. Visualization of samples was performed using a Dragonfly SLite 140 transilluminator. For quantification and purity assessment, samples were analysed spectrophotometrically with a NanoDrop ND-1000 system (Thermo Fisher Scientific). When necessary, desired DNA fragments were purified from gels with the NZYGelpure kit.

### DNA amplification and cloning

Amplification of desired DNA fragments was performed using specific pairs of primers (TABLE 1) synthesized at IDT, on either a 2720 Thermal Cycler (Applied Biosystem) or a LabnetTM MultigeneTM OptiMax (LabnetTM) thermocyclers. Routine PCRs were performed using NZYTaq II DNA Polymerase. When higher fidelity, reliability or speed was required, High-Fidelity Phusion or Q5 DNA Polymerases were used. The specific annealing temperatures of each pair of primers were calculated with the NEB Tm calculator tool [https://tmcalculator.neb.com].

PCR products containing the coding sequences (CDSs) of the proteins produced in this study were amplified from gDNA or synthetic DNA fragments using high fidelity Phusion DNA polymerase. The PCR annealing temperatures were calculated (TABLE 1) and the extension time was 30-60 s. NZYTaq II DNA polymerase was used to add adenine nucleotides to the blunt ends of the amplified DNA fragments, which were then gel purified and cloned into the pre-linearized pGEM-T Easy vector.

An in vivo assembly cloning (IVA Cloning) strategy (Garcia-Nafria, J., Watson, J. F., & Greger, I. H. (2016). IVA cloning: A single-tube universal cloning system exploiting bacterial in vivo assembly. Scientific Reports, 6, 27459. https://doi.org/10.1038/srep27459) was employed. This procedure involves the in vivo homologous recombination of two PCR amplicons: the amplified linear plB4 vector and the amplified coding sequence of interest, which was flanked by the pIB4 recombination sequences included in the designed oligonucleotides (TABLE 1). The high fidelity Q5 DNA polymerase was used. The conditions for the pIB4 amplification were: (i) 98 °C for 30 s; (ii) 30 cycles of 98 °C for 10 s, 65 °C for 30 s, 72 °C for 240 s; (iii) 72 °C for 120 s. The CDSs amplification followed similar conditions but with an annealing temperature indicated in the referred tables and an extension time of 45 s. The PCR products were gel purified and mixed at a 1:5 vector-to-insert ratio (v/v).

**TABLE 1. Oligonucleotides used for amplification and cloning of hyaluronidase CDS.**

| **Primer** | **Anneali ng (°C)** | **Sequence (5'-3')** | **Descript ion** |
|---|---|---|---|
| **PLYA44 *1*(+)** | 68 | ATGAAGTTGCTGACTACCTTGCCAGC (SEQ ID No.12) | *pILya44* CDS amplificat ion |
| **PLYA44 *1*(-)** | | TTACTCAGCGTGGGTCAAAGTTTCC (SEQ ID No.13) | |
| **pIB4(+)** | 65 | **GAATTCGAGCTCGGTACCC** (SEQ ID No.14) | pIB4 amplificat ion |
| **pIB4(-)** | | **AGCTTCAGCCTCTCTTTTCTC** (SEQ ID No.15) | |
| **PLYA44 *2*(+)** | 66 | GGTATCTCTCGAGAAAAGAGAGGCTGAAGCTGCTCCACAA AGAGAGAACGCTC (SEQ ID No.16) | *pILya44* CDS IVA cloning |
| **PLYA44 *2*(-)** | | ATCCCCGGGTACCGAGCTCGAATTCTTACTCAGCGTGGGT CAAAGTTTC (SEQ ID No.17) | |

| | | | |
|---|---|---|---|
| Regions annealing with pIB4 sequences are shown in bold. | | | |

### Colony PCR and sequencing

Colony PCRs were performed to screen the presence or absence of the DNA fragments of interest in plasmid (*E*. *coli*) or genomic DNA (*P*. *pastoris*)*.* Thus, individual transformants were lysed with a 10 min heating step (bacteria) or alternating 10 s cycles of heating-cooling (yeasts, 10-15 times), respectively. Samples were centrifugated (13,000 rpm, 5 min), and the supernatants (2-3 µL) then used as templates in routine PCRs. The integrity of the generated constructs was verified by DNA sequencing at Macrogen. The oligonucleotides employed in colony PCRs and sequencing are indicated in TABLE 2.

**TABLE 2. Oligonucleotides used in colony PCR and sequencing.**

| **Primer** | **Annealing (°C)** | **Sequence (5'-3')** | **Description** |
|---|---|---|---|
| **AOX(+)** | 55 | GACTGGTTCCAATTGACAAGC (SEQ ID No.18) | Colony PCR and sequencing pIB4 vector |
| **AOX(-)** | | GCAAATGGCATTCTGACATCC (SEQ ID No.19) | |
| **T7P(+)** | 50 | TAATACGACTCACTATAGG (SEQ ID No.20) | |
| **SP6(-)** | ATTTAGGTGACACTATAGA (SEQ ID No.21) | Colony PCR and sequencing pGEM-T vector | |

### Plasmid DNA linearization

The final pIB4 derivative constructs (10-30 µg) were linearized at the *HIS4* locus using FastDigest Sall restriction enzyme prior to transformation of *P*. *pastoris.* After that, linearized DNA samples were precipitated by adding 1/10 volumes of 3 M sodium acetate and 2 volumes of cooled absolute ethanol. The DNA was pelleted by centrifugation at 13,000 rpm for 15 min at 4 °C, and then resuspended in 10 µL of sterile water.

### Obtention and analysis of the fungal secretome

*Purpureocillium* species were cultured in 200 mL of HAI medium supplemented with 0.2-2 g/L of HMW HA for 48-96 h. The hyaluronidase activity at specified culture times was assessed as detailed in the previous section. The optimal culture time for inducing HA-degrading activity was determined and used as the incubation time for subsequent cultures. For size exclusion chromatography (SEC) purification, the culture of *Purpureocillim sp.* showing HA-degrading activity was scaled up to 1 liter. The extracellular protein fraction was collected by centrifugation at 6,000 rpm for 45 min at 4 °C.

### Heterologous protein expression in Pichia pastoris:

### Recombinant protein production in flask cultures

The selected *P. pastoris* colonies integrating the desired pIB4 derivative constructs were inoculated from MD plates into 250 mL flaks containing 25 mL of BMG medium. The cultures were grown for 16-18 h until they reached an OD600 of 4.0-8.0. Cells were harvested by centrifugation at 2,000 rpm for 5 min at 4 °C using a 5810/5810R Benchtop Centrifuge (Eppendorf), then transferred to 1 L flaks containing the appropriate volume of BMM medium to achieve an initial OD600 of 1.0-2.0. The cultures were incubated for 120 h and the induction of protein expression was conducted by adding 0.5 % methanol every 24 h. The growth of the cultures was periodically monitored, and the pH was maintained at 6.0 using 1 M potassium phosphate buffer, if necessary. Colonies of P. pastoris containing the plB4 empty vector were used as negative controls. Cells were subsequently removed by centrifugation at 2,000 rpm for 10 min at 4 °C.

### Recombinant protein production via fed-batch fermentation

A 5-L Biostat B Plus bioreactor (Sartorius) was used to scale up the production of selected biocatalysts. The P. pastoris transformants were initially cultivated in 100 mL of BMG medium for 48 h, then used to inoculate the bioreactor containing 2 L of FBS medium, achieving an initial OD600 of approximately 0.004. The fermentation parameters were maintained as follows: 30 °C, 1,000 rpm agitation, 20 % dissolved O2, and pH controlled at 6.0 for 138 h. Methanol was added over 3 days at a rate of 2.7-4.6 mLVh/L, resulting in a final culture OD600 of about 150. Cells were subsequently removed by centrifugation at 5,500 rpm for 15 min at 4 °C.

### Proteomic analysis

In-gel digestion, reverse-phase (RP) liquid chromatography (LC) and tandem-mass spectrometry (MS/MS) analyses were carried out in the CBMSO Protein Chemistry Facility. Three distinct analyses were conducted to identify peptides from (i) fungal secretomes, (ii) specific gel bands, or (iii) activity spots in zymography.

Extracellular fractions of fungal cultures were concentrated and loaded into 1.2-cm wide wells of a 12 % SDS-PAGE. The electrophoresis run was halted as soon as the dye front migrated 3 mm into the resolving gel, concentrating the entire secretome at the stacking/resolving gel interface. The unseparated protein bands were stained, excised, cut into 2x2 mm cubes, and placed in 0.5 mL microcentrifuge tubes (Eppendorf). The gel pieces were destained in a 1:1 ACN:H₂O solution, followed by reduction of disulphide bonds with 10 mM DTT for 1 h at 56 °C (for secretome analysis) or 60 °C (for gel bands or activity spots). Thiol groups were then alkylated with 10 mM iodoacetamide for 30 min in darkness. In situ digestion was performed using sequencing-grade trypsin, following a protocol adapted from Shevchenko, A., Wilm, M., Vorm, O., & Mann, M. (1996). Mass spectrometric sequencing of proteins from silver-stained polyacrylamide gels. Analytical Chemistry, 68(5), 850-858. https://doi.org/10.1021/ac950914h with minor modifications. The gel pieces were dehydrated by removing all liquid with ACN, following by drying in a speedvac. They were then rehydrated in Tris-HCl 100 mM pH 8.0 and 10 mM CaCl₂ buffer containing 60 ng/µL trypsin at 5:1 protein-to-enzyme (w/w) ratio. The tubes were kept on ice for 2 h, followed by incubation at 37 °C for 12 h. Digestion was halted by adding 1 % TFA (w/v). The supernatants were dried down and desalted using ZipTip C18 pipette tips (Millipore) prior to mass spectrometric analysis.

The desalted and dried protein digest was resuspended in 10 µL of 0.1 % formic acid (v/v) and analysed by RP-LC-MS/MS using an Easy-nLC II system coupled to an LTQ-Orbitrap-Velos-Pro hybrid mass spectrometer (Thermo Fisher). Peptides were concentrated using a 0.1 mm × 20 mm C18 RP precolumn (Thermo Fisher) and then separated on a 0.075 × 250 mm C18 RP column (Thermo Fisher) at a flow rate of 0.3 µL/min. Peptides were eluted using a 90-min dual gradient: 5-25 % solvent B for 45 min, 25-40 % solvent B for 15 min, 40-100 % solvent B for 2 min and 100 % solvent B for 18 min (solvent A: 0.1% formic acid in water, solvent B: 0.1% formic acid, 80% ACN in water). ESI ionization was performed using a Stainless-Steel ID 30 µm interface Nano-bore emitter (Proxeon) at 2.1 kV spray voltage with an S-Lens setting of 60 %. The Orbitrap resolution was set at 30,000. Peptides were detected in survey scans ranging from 400 to 1,600 amu (1 µscan), followed by 20 data-dependent MS/MS scans (Top 20), using a 2 u isolation width (in m/z units), 35 % normalized collision energy, and dynamic exclusion applied for 60 s. Charge-state screening was enabled to exclude unassigned and singly charged ions.

For secretome analysis, when the sequence of a peptide of interest was known, the mass spectrometer was operated in selected MS/MS ion monitoring (SMIM) mode (Jorge, I., Casas, E. M., Villar, M., Ortega-Pérez, I., López-Ferrer, D., Martinez-Ruiz, A., Carrera, M., Marina, A., Martinez, P., Serrano, H., Benito-Cañas, F., Were, J. M., Gallardo, S., Lamas, J. M., Redondo, D., Garcia-Dorado, D., & Vázquez, J. (2007). High-sensitivity analysis of specific peptides in complex samples by selected MS/MS ion monitoring and linear ion trap mass spectrometry: Application to biological studies. Journal of Mass Spectrometry, 42(11), 1391-1403. https://doi.org/10.1002/jms.1314). In this mode, the LTQ-Orbitrap-Velos-Pro was programmed to continuously perform sequential MS/MS operations on doubly or triply charged ions corresponding to peptides selected from theoretical predictions.

Peptide identification from raw data was carried out using PEAKS Studio Xpro search engine (Bioinformatics Solutions Inc.). Database searches were conducted against the uniprot-purpureocillium-lilacinus.fasta (Proteome ID: UP000078340, 11,750 entries; UniProt release 07/2023) with a decoy-fusion database. The search parameters included tryptic cleavage after Arg and Lys (semi-specific), allowance for up to two missed cleavage sites, and mass tolerances of 20 ppm for precursor ions and 0.6 Da for MS/MS fragment ions. The searches also accounted for optional modifications such as Met oxidation and Cys carbamidomethylation. False discovery rates (FDR) for peptide spectrum matches (PSM) and for protein were limited to 0.01. Only proteins identified by at least two unique peptides from the RP-LC-MS/MS analyses were considered reliable.

### Hyaluronic acid degradation

The assay used 2.5-5.0 g/L of hyaluronate in the presence of 0.01-0.1 mg/mL of PILya44 enzyme or 10 µL of extracellular enzymatic solution. To monitor the enzymatic reactions, aliquots were taken at various time points, boiled for 10 min and diluted 1:10 (v:v) with MilliQ water before the analysis.

The compounds were eluted by a gradient method at a flow rate of 0.5 mL/min in which the initial mobile phase was 30 mM NaOH and 270 mM of sodium acetate trihydrate for 45 min, then from minute 45 to minute 70, the mobile phase was increased gradually until it reached a concentration of 100 mM NaOH and 900 mM of sodium acetate, which was further maintained during 5 min. Finally, initial conditions were reset and maintained for 10 min to equilibrate the column.

### Mass spectrometry analysis

HAOS mixtures were analysed using matrix-assisted laser desorption/ionization time-of-flight (MALDI-TOF) mass spectrometry (MS) on an Ultraflex III TOF/TOF (Bruker) instrument equipped with a neodymium-doped (Nd) laser. Samples were mixed with 20 µL of matrix solution (10 mg/mL 2,5-dihydroxybenzoic acid) in CH₂OH with 10 % H₂O. To reduce salt content, which can inhibit ionization and increase the number of signals for the same compound if present in different salt forms (e.g., carboxylic acids mixed with sodium carboxylates), a spatula tip of strong cation exchange resin was added. Spectra were recorded in reflector mode, detecting both positive and negative ions. The acquisition range spanned from m/z 50 to 5,000 Da.

### Large-scale production of HAOS

A method was developed for the large-scale production of HAOS mixtures using HMW HA. Hyaluronate solutions were prepared at a concentration of 2.5 g/L (w/v) in distilled water. The degradation of HA was carried out using PILya44 enzyme (0.01 mg/mL). Incubation was carried out at 50 °C with shaking at 250 rpm for durations of 3 hours and 72 hours. The reactions, with a total volume of 0.2 L, were terminated by immersion in a boiling water bath for 15 min, followed by filtration through 0.45 µm filters. Subsequently, the enzymes were removed via ultrafiltration using a Vivaflow^{®} system equipped with a 30 kDa molecular weight cutoff membrane. All samples were freeze-dried prior to analysis. The resulting HAOS mixtures were characterized using HPAEC-PAD and mass spectrometry, as previously described in *Hyaluronic acid degradation* section and *Mass spectrometry analysis* section, respectively.

### Protein purification, concentration, and buffer exchange

The extracellular media from the fungal and yeasts cultures were filtered sequentially through glass fibre GF/C filters (Whatman) and nitrocellulose membranes with pore sizes of 0.45 µm and 0.22 µm (Merck). If necessary, extracellular fractions of *P*. *pastoris* cultures were concentrated by tangential flow filtration using Vivaflow^{®} 50 cassettes with a 30 kDa cutoff (Sartorius) to reduce the volume to approximately 50 mL. The extracellular fraction of fungal cultures was concentrated via pressure driven filtration to about 10-20 mL, using 50 and 200 mLAmicon^{®} Stirred Cells with 10 kDa membranes (Millipore), used in conjunction with a compressed N2 source and a magnetic stirrer. Further concentration to volumes of 0.5-2 mL was performed using 10 or 30 kDa Vivaspin^{®} filters (Sartorius). Additionally, concentrated proteins were subjectedto buffer exchange via diafiltration using Vivaspin^{®} filters using Tris-HCl 20 mM pH 6.0-7.0 (*P*. *pastoris* cultures) and pH 6.0-8.0 (fungal cultures).

### Size exclusion chromatography

Protein purification from the fungal secretome was achieved using preparative size exclusion chromatography on an ÄKTA pure^{™} chromatography system (Cytivia). The dialyzed protein concentrate was filtered through a 0.22 µm membrane to ensure clarity and remove any remaining small contaminants. Subsequently, the sample was loaded onto a HiLoad 26/600 Superdex 200 pg column (Cytiva), pre-equilibrated with Tris-HCl buffer (20 mM, pH 8.0). The proteins were separated based on size at a flow rate of 0.8 mL/min. Collected fractions were stored at 4 °C to preserve stability until further activity assays could be conducted.

### Polyacrylamide gel electrophoresis

### SDS-PAGE

Protein samples were analysed by denaturing and reducing sodium dodecyl sulphate polyacrylamide gel electrophoresis (SDS-PAGE) using a discontinuous buffer system, following the protocol established by Laemmli (1970). The resolving gels were composed of 12-15 % (v/v) polyacrylamide, while 3 % polyacrylamide stacking gels were cast on top to ensure optimal protein resolution. Electrophoresis was performed in a Mini-Protean 3 vertical system (Bio-Rad) using Tris-Glycine buffer under the following conditions: 180 V, 4 °C, 60 min. Prior to electrophoresis, protein samples were denatured by heating for 10 min in a sample buffer containing 1.0 % SDS (w/v), 4.2 % glycerol (v/v), 5.0 % BME (w/v), 2.5 % DTT (w/v), 0.01 % BPB (w/v) in Tris-HCl 31 mM pH 6.8. After separation, the gels were stained with BlueSafe. Precision Plus Protein Standards Unstained 10-250 kDa were used as molecular weight markers.

### In-gel zymography

Hyaluronidase activity in SEC purified protein samples was detected using zymography, following a protocol adapted from Velesiotis, Vasileiou & Vynios (2019), with minor modifications. Gels consisting of 10% polyacrylamide were prepared, into which 0.5 g/L HMW HA was entrapped. The stacking and resolving gels were cast without SDS. The BTH (0.3 U/mg) was used as positive control, and the BSA (0.4 mg/mL) served as molecular weight marker. Protein samples were mixed with a sample buffer containing 1.0 % SDS (w/v), 4.2 % glycerol (v/v), 0.01 % BPB (w/v) in Tris-HCl 31.2 mM pH 6.8. Electrophoresis was carried out under the following conditions: 100 V, 4 °C, 120 min. Once the electrophoresis was completed, the gels were transferred to a container with solution 1 % Triton-X in water (v/v) for 30 min to remove SDS. The gels were then incubated in distilled water at 45 °C for 1-2 h with gentle shaking, following by staining for 30 min in a solution containing 0.5 % (w/v) Alcian Blue 8GX in 3 % (v/v) acetic acid. Excess of dye was removed by sequential washing in two destaining solution: one containing 7 % of acetic acid and the second containing also 50 % methanol (both v/v).

### Protein quantification

Protein levels were measured at 280 nm using a Nanodrop ND-1000 Spectrophotometer version 3.8 (Thermo Fisher Scientific) or quantified by densitometry of SDS-PAGE resolved gels, analysed with imaged Software version 1.53t.

### Antioxidant activity

The antioxidant activity of various compounds was assessed using the ABTS radical cation (ABTS·+) discoloration method, as described by Re et al. (1999), with modifications for 96-well plates. The ABTS·+ was generated by mixing equal volumes of a 7 mM ABTS solution and a 2.45 mM potassium persulfate (K2S2O8) solution in Milli-Q water. This mixture was incubated in the dark overnight at room temperature. Following incubation, the solution was diluted with Milli-Q water to obtain an absorbance of 0.7 units at 655 nm.

Several compounds were tested at different concentrations: from 0 to 150 mg/mL for obtained hyaluronan oligosaccharides, HyaloBooster, and HyaloOligos and from 0 to 5 mg/mL for HMW HA. For each concentration, 20 µL of the sample was added to the wells of a 96-well plate in triplicate, followed by the addition of 230 µL of the ABTS·+ solution. The microplate was incubated in the dark at room temperature for 15 minutes. Absorbance was then measured at 655 nm using a microplate spectrophotometer (Versamax, Molecular Devices). Trolox was used as a reference antioxidant and was tested across a range of concentrations from 0 to 600 µM in methanol. The SC50 value was defined as the concentration of the compound required to reduce the absorbance of the ABTS·+ by 50 %. All samples were measured in triplicate, and the data were expressed as the mean ± standard deviation.

### Statistical analyses

All statistical analyses were performed using GraphPad Prism software version 9.0. Data were first tested for normality using the Shapiro-Wilk test, which indicated a significant departure from normality (W(18) = 0.5619, p < 0.001) of SC50 values. As the SC50 values for antioxidant activity did not meet the assumptions of normality, non-parametric statistical methods were applied.

A Kruskal-Wallis test was used to compare the SC50 values of HMW HA, HyaloOligos, HyaloBooster, HAOS-3h, HAOS-72h, and Trolox. This non-parametric test, which is the equivalent of a one-way ANOVA but does not assume normal distribution, was applied to compare the medians across the referred independent groups. The Kruskal-Wallis test results were expressed as a test statistic (H) and associated p-value, with significance set at p < 0.05. A Dunn's multiple comparisons test was performed to identify specific pairwise differences. Dunn's test is a rank-based multiple comparison method that accounts for the number of groups tested and adjusts the p-values accordingly to control for type I errors. Adjusted p-values were reported, and significance thresholds were defined as follows: p < 0.05 (*), p < 0.01 (**), p < 0.001 (***), and p < 0.0001 (****). Comparisons that were not statistically significant were indicated as "ns".

### Results

### Identification of a hyaluronidase-producing microorganism and characterization of its hyaluronate-degrading activity

The hyaluronidase activity of various fungal strains evaluated using a qualitative plate assay (Patil, S., & Chaudhari, B. (2017). A simple, rapid and sensitive plate assay for detection of microbial hyaluronidase activity. Journal of Basic Microbiology, 57(4), 358-361. https://doi.org/10.1002/jobm.201600579), as outlined above in the *filamentous fungi* section. The filamentous fungus *Purpureocillium sp.* demonstrated the highest hyaluronidase activity, evidenced by a pronounced clearance zone resulting from the depolymerisation of HMW HA.

Previous attempts to induce significant hyaluronidase activity in *Purpureocillium sp.* cultures in YEPD medium were unsuccessful. Consequently, the fungus was cultivated in HAI medium, supplemented with 0.2 g/L HMW HA as the primary carbon source. This approach was designed to selectively induce the expression of HA-degrading enzymes during a 96-hour cultivation period. The medium composition was adapted from protocols previously used for the *Penicillium spp.* hyaluronidase expression, replacing glucose with HA as the carbon source (Bakke, M., Kamei, J. I., & Obata, A. (2011). Identification, characterization, and molecular cloning of a novel hyaluronidase, a member of glycosyl hydrolase family 16, from Penicillium spp. FEBS Letters, 585(1), 115-120. https://doi.org/10.1016/j.febslet.2010.11.021). Due to the flocculation level observed during fungal growth, which hindered accurate optical density measurements, the cultures were inoculated with a consistent 0.1 g of wet cellular mass.

To monitor HA degradation, extracellular fractions from the fungal cultures were collected, processed, and analysed via HPAEC-PAD (Section *Hyaluronic acid degradation* above). This method, enabled efficient separation of HAOS (FIG 1). The elution profile of the oligosaccharides aligned with their increasing DP, as it was reported. This technique provides sensitive detection of oligosaccharides and LMW HA fragments ranging from 1 to 25 dimeric β-GIcA(1,3)-β-GIcNAc(1,4) HA units. The presence of HAOS confirmed the enzymatic degradation of HA, thereby verifying the successful induction of hyaluronidase expression and activity in *Purpureocillium sp.* cultures. To evaluate the progression of HA-degrading activity, hyaluronidase activity in extracellular fractions collected at different time points during cultivation was quantified. The enzymatic assays were performed at 30 °C and 900 rpm, using 0.25 g/L HMW HA as the substrate, prepared in 0.1 M sodium acetate buffer (pH 6.0). After a 1-hour reaction period, the generation of reducing sugar ends was quantified using the BCA method (*Hyaluronidase activity* section). Maximum hyaluronidase activity was measured at 48 hours of cultivation (FIG 2).

After selecting a 48-hour incubation period to achieve significant expression of HA-degrading activity, the optimal pH and temperature for the degradation of HMW HA were determined using as enzymatic solution the fungal extracellular fraction obtained by the culture diafiltration, as outlined in Section *Protein purification, concentration, and buffer exchange* of Materials and Methods. The optimal conditions were found to be pH 4.0 and 50 °C. Reported optimal conditions for fungal extracellular hyaluronidases vary widely: pH 4.0 and 20 °C for hyaluronate lyase from *F. hepatica;* pH 3.0 and 40 °C for hyaluronidase from *Talaromyces stipitatus* (Bobkova, L., Smirnou, D., Krcmar, M., Kulhanek, J., Hermannova, M., Franke, L., & Velebny, V. (2016). Discovery and characteristic of hyaluronidases from filamentous fungi. Current Biotechnology, 7(1), 2-9. https://doi.org/10.2174/2211550105666160213002658); pH 6.0 and 37 °C for hyaluronate lyase from *C*. *laurentii* DSMZ 70766; pH 3.0 and 37-45 °C for hyaluronidase from *Pseudozyma aphidis* (Smirnou, D., Krčmář, M., Kulhánek, J., Hermannová, M., Bobková, L., Franke, L., Pepeliaev, S., & Velebný, V. (2015). *Characterization of hyaluronan-degrading enzymes from yeasts.* Applied Biochemistry and Biotechnology, 177(3), 700-712. https://doi.org/10.1007/s12010-015-1774-0); and pH 3.0-4.0 and 40-45 °C for hyaluronidase from *Talaromyces purpureogenus* (Bakke et al., 2011). Notably, in all these cases, including the HA-degrading activity from *Purpureocillium sp.,* the enzyme activities were measured directly from extracellular fractions of fungal culture filtrates or semi-purified enzymatic extracts. The hyaluronidase activity characterized in this study exhibits a distinctive profile, with optimal activity at a higher temperature (50 °C) and slightly acidic pH (4.0), making it potentially suitable for applications requiring stable and efficient enzymatic degradation under these conditions.

### Purification and identification of a novel hyaluronidase

The initial step involved characterising the protein composition of the secretome from *Purpureocillium lilacinum.* cultivated in HAI medium. The fungal culture was harvested after 48 hours of growth. The extracellular fraction was dialysed and concentrated to a final protein concentration of approximately 0.1 mg/mL, as described in *Obtention and analysis of the fungal secretome* of the Materials and Methods. To analyse the protein composition of the secretome, a shotgun proteomic approach was employed, which included in-gel digestion, reverse-phase liquid chromatography, and tandem mass spectrometry, as detailed in *Proteomics* section of Materials and Methods.

RP-LC-MS/MS analysis led to the identification of 652 peptides and their corresponding protein set. Database searches were performed using the *P*. *lilacinum* proteome (Proteome ID: UP000078340, 11,750 entries; UniProt release 07/2023), resulting in the identification of 43 distinct proteins, ranging from 14 to 120 kDa in size. Functional analysis via InterPro annotations revealed several hypothetical polysaccharide lyase enzymes (UniProt IDs: A0A179HJB3, A0A179GQM7, A0A179H1L8, and A0A179GC80). However, none of these proteins were clearly assigned to polysaccharide lyase families known to degrade hyaluronate. In fact, the dbCAN3 annotation server classified then in the GH136 (HMMER E-value=4.7·10-84), no assignment, GH55 (HMMER E-value=1.3-10-129) and GH28 (HMMER E-va!ue=6.4 10-59), respectively.

At this stage, a protein purification step using size-exclusion chromatography was performed. Chromatography was carried out as outlined above. During elution, 1 mL aliquots were collected, and each was analysed for HA-degrading activity. The activity of each fraction was assessed in a 4-hour reaction using HMW HA as substrate, followed by HPAEC-PAD analysis. HA-degrading proteins eluted between 225 and 230 mL, and their activity was confirmed by HPAEC-PAD. Furthermore, the analysis of the degradation products was performed using MALDI-TOF MS in both ionisation modes. Even-numbered unsaturated HAOS with DP between 1 and 6 dimeric HA units were detected, with the unsaturated dimer being the smallest product.

The fraction exhibiting HA-degrading activity was further analysed via SDS-PAGE (FIG 3.A), and the main protein band, approximately 40 kDa in size, was subjected to RP-LC-MS/MS analysis, as described above. A total of 143 peptides and 10 proteins were identified. Database searches were consistent with the secretome analysis, identifying a protein of 44 kDa (UniProt ID: A0A179GQM7), with high sequence coverage (57 %) and 30 unique peptides. Additionally, 9 contaminant proteins, ranging from 11 to 57 kDa, were detected with varying levels of sequence coverage. This 44 kDa protein had previously been identified in the fungal secretome analysis and was annotated as a lyase-type enzyme, potentially acting on chondroitin or alginate substrates UniProt ID: AOA179GQM7.

To confirm the role of this potential protein in HA degradation, in-gel zymography analysis under semi-native conditions was performed as a complementary method, as described above. In-gel zymography offers several advantages for detecting enzyme activity, including the simultaneous determination of molecular weight and activity, high sensitivity, and the preservation of the native or partially native state of the enzyme, enabling activity-based detection (Velesiotis, C., Vasileiou, S., & Vynios, D. H. (2019). Analyzing hyaluronidases in biological fluids. In Methods in Molecular Biology (Vol. 1952, pp. 127-142). Springer. https://doi.org/10.1007/978-1-4939-9133-4_12). The BSA was used as a molecular weight marker and the native BTH as a positive control of hyaluronidase activity (FIG 3.B, lanes 2 and 3, respectively). HA-degrading activity was visualised using Alcian Blue 8GX dye, as depolymerisation of HA resulted in unstained zones. An activity spot below 55 kDa (as indicated the activity spot of BTH, FIG 3.B, lane 3) was observed and analysed via RP-LC-MS/MS. Additionally, an unstained zone at the top of the resolving gel was identified and also analysed (FIG 3.B, lane 1).

RP-LC-MS/MS analysis of both samples yielded good-quality fragmentation spectra. The 44 kDa protein (UniProt ID: A0A179GQM7) was identified again, albeit with lower sequence coverage (13 %) and 5 unique peptides.

In summary, the analysis of the *Purpureocillium lilacinum* CECT 28398T secretome has revealed a 44 kDa potential lyase-type enzyme. This protein likely exhibited promising HA-degrading activity, as demonstrated by a combination of chromatographic, electrophoretic, and mass spectrometric methods. The consistent identification of this enzyme, along with the detection of HAOS, highlights its potential involvement in HA degradation.

### Heterologous expression and functional validation of a novel hyaluronidase

Following its identification and preliminary characterization, the next step was to heterologously express and functionally validate the potential HA lyase. This would enable a deeper understanding of its catalytic properties and potential applications in biotechnological processes. The sequence of the protein (encoding 399 amino acids), now referred to as PILya44 (UniProt ID: A0A179GQM7), revealed a putative signal peptide with a predicted protease cleavage site between residues 21 and 22, showing a high probability of 0.999.

The already annotated DNA sequence of PILya44 (NCBI ID: XM_018324517.1) was optimized for codon usage in *P*. *pastoris* GS115 and synthesized as a string DNA fragment (Section 3.2.3.3). Specific primer pairs (TABLE 1, Materials and Methods) were designed and used in PCR reactions, as detailed in Section *DNA amplification and cloning* of the Materials and Methods. This enabled the amplification of the pllya44 hyaluronidase coding sequence, which was then cloned into the pGEM-T vector (Promega), resulting in the construct pGEM-PILya44 (4.2 kb; *E. coli* as host; ampicillin resistance marker, α-peptide coding region of β-galactosidase). Subsequently, the CDS (SEQ ID No. 5), excluding the signal peptide, was cloned into the plB4 vector, where it was fused in-frame with the aMF sequence, generating the construct pIB4-PILya44 (7.2 kb; *E. coli*/*P. pastoris* as hosts; ampicillin resistance marker, histidinol dehydrogenase 4 wild-type allele; alcohol oxidase 1 promoter; and *Saccharomyces cerevisiae* α-mating factor secretion signal sequence) the plB4 vector comprising the aMF sequence was disclosed in Gimeno-Pérez, Maria & Linde, Lola & Fernandez-Arrojo, Lucia & Plou, Francisco & Lobato, Maria. (2014). Heterologous overproduction of β-fructofuranosidase from yeast Xanthophyllomyces dendrorhous, an enzyme producing prebiotic sugars. Applied microbiology and biotechnology. 99. 10.1007/s00253-014-6145-1. An in vivo assembly cloning strategy was employed to create this construct (see Section *DNA amplification and cloning*), using specific primer pairs (TABLE 1). The construct was linearized at the HIS4 locus and transformed into *P*. *pastoris* (see *Plasmid DNA linearization* Section and *Yeasts transformation* Section). Successful transformation was confirmed by colony growth on MD medium without histidine, and sequence integration was validated through colony PCR with AOX(+) and AOX(-) primers (TABLE 2, Section *Colony PCR and sequencing* of Materials and Methods).

PILya44 expression in the selected P. *pastoris* transformant was performed in BMM medium (see Section *Recombinant protein production in flask cultures*)*.* Hyaluronidase activity was measured using HMW HA as the substrate (FIG 4), with the highest activity of 0.62 U/mL detected in the extracellular fraction after 120 hours of cultivation. As expected, the negative control transformant with the empty plB4 vector showed no detectable hyaluronidase activity (FIG 4, empty circles).

The extracellular PILya44 protein produced by *P. pastoris* at different cultivation times was analysed in SDS-PAGE (FIG 5). With a theoretical molecular mass of 42.1 kDa, PILya44 appeared as the major and almost unique protein band of the extracellular fraction, reaching a maximum concentration of 0.07 g/L after 120 hours of cultivation (FIG 5.A). The higher molecular weight smear likely indicated the partial glycosylation of the protein. To address this, a 24-hour deglycosylation treatment with EndoH under denaturing conditions was performed, which reduced the size of the major protein band and clearly eliminated the smear (FIG 5.B).

This analysis unequivocally assigns hyaluronidase-type activity to the previously identified protein sequence from the secretome, underscoring its potential role in biotechnological applications. The successful expression and functional validation of PILya44 pave the way for applications in enzyme-based processes.

### Enzymatic characterization of PILya44 hyaluronidase: optimal reaction conditions, stability, and kinetics

The hyaluronidase activity of PILya44 was assessed to determine the optimal conditions for enzymatic assays using HMW HA as the substrate (FIG 6). The enzyme exhibited maximum activity at temperatures between 45-50 °C (FIG 6.A) and pH 4.0 (FIG 6.B).

PILya44 shows unique optimal conditions, particularly its ability to function at higher temperatures and more acidic pH compared to most bacterial HA lyases.

PILya44 hyaluronidase showed notable thermal stability. After 1 h of incubation at 60 °C maintained 50 % of its activity (FIG 6.C), compared to many other hyaluronidases. The marked thermal stability of PILya44 sets it apart from most bacterial and animal hyaluronidases, which typically lose activity at lower temperatures, making it highly suitable for industrial applications requiring prolonged enzyme function at elevated temperatures. This robustness positions PILya44 as a versatile enzyme for diverse biotechnological processes that demand high-temperature resistance. These properties make it a promising enzyme for specialized applications requiring HA degradation under such conditions.

Additionally, the activity of PILya44 was negatively affected by the presence of metal ions, with significant inhibition observed when divalent ions like Zn²⁺ were present (FIG 6.D). This is in opposition to some bacterial hyaluronate lyases, such as those from *Arthrobacter globiformis* and *S*. *pyogenes*, which are stimulated by divalent metal ions like calcium (Ca²⁺) and magnesium (Mg²⁺), respectively (Singh et al., 2014; Zhu et al., 2017). This feature is relevant for industrial purposes, because unlike bacterial hyaluronidases, the enzyme of the invention does not require the addition of divalent ions to achieve the maximum activity, thus this enzyme use can reduce industrial costs. The PILya44 enzyme demonstrated high activity against HMW HA but exhibited limited activity against CS (TABLE 3), and no activity against alginate substrate.

**TABLE 3. Substrate specificity of PILya44 hyaluronidase.**

| **Substrate** | **Activity (%)** |
|---|---|
| HMW HA | 100.0 ± 1.7 |
| Chondroitin sulphate | 35.1 ± 0.2 |
| Dermatan sulphate | n.a.d. |
| Heparin | n.a.d. |
| Colloidal chitin (α-chitin) | n.a.d. |
| Pectin | n.a.d. |
| Alginate | n.a.d. |
| Laminaran | n.a.d. |

Values are the percentages of the maximum activity assayed (0.3 U/mL, measured with HMW HA). Activity data are the average of three assays and standard errors are indicated. n.a.d., no activity detected.

It was unable to degrade highly sulphated or structurally complex GAGs, such as heparin and dermatan sulphate. The lack of activity against unrelated polysaccharides, including chitin, alginate, laminarin, and pectin, is attributed to their significantly different chemical structures and linkage types (see TABLE 4).

**TABLE 4. Characteristic of the carbohydrates employed**

| **Polysaccharides** | | | | |
|---|---|---|---|---|
| **Carbohydrate** | **Description** | **Structure** | **Manufacturer** | |
| **Hyaluronic acid from *Streptococcus equi*** | 1500-1800 kDa (High molecular weight) | [β-GlcA(1,3)-β-GlcNAc(1,4)]ₙ | Sigma-Aldrich | |
| **Sodium chondroitin sulphate** | - | [β-GlcA(1,3)-β-GalNAc-4/6S-(1,4)]ᵣ | Biosynth | |
| **Sodium alginate** | - | [β-ManA(1,4)-α-GulA(1,4)]ₙ | Sigma-Aldrich | |
| **Pectin from citrus peel** | GaIA ≥ 74 %; Methoxy Groups ≥ 6.7 % | | [β-GaIA(1,4)]ₙ | Sigma-Aldrich |
| **Laminaran from *Eisenia bicyclis*** | - | | [β-D-Glc(1,3)]ₙ | Biosynth |
| **Dermatan sulphate sodium salt** | - | | [β-IdoA(1,3)-β-GaINAc-4/6S-(1,4)]ₙ | Sigma-Aldrich |
| **Hyaluronic acid tetrasaccharide** | | ΔHexA-β(1,3)-GlcNAc-β(1,4)-GlcA-β(1,3)-GlcNAc | | Iduron |
| **HyaloBooster ≤ 2 kDa** | | ΔHexA-β(1,3)-[β-GlcNAc(1,4)-β-GlcA(1,3)]ₘ | | Kewpie Corporation |
| **HyaloOligos ≤ 10 kDa** | | [β-GlcA(1,3)-β-GlcNAc(1,4)]ₘ | | Kewpie Corporation |

HA lyases from different sources exhibit different substrates specificities. For instance, that from S. *hyalurolyticus* is HA-specific and that from *Stenotrophomonas maltophila* displays also activity against alginate (Sindelar, M., Jilkova, J., Kubala, L., Velebny, V., & Turkova, K. (2021). Hyaluronidases and hyaluronate lyases: From humans to bacteriophages. Colloids and Surfaces B: Biointerfaces, 208, 112095. https://doi.org/10.1016/i.colsurfb.2021.112095.; and Stern & Jedrzejas, 2006). Kinetic analysis indicates that PILya44 exhibits a substantial preference for HMW HA over CS, with an apparent *k*_{cat}/*K*ₘ ratio that showed it degrades HMW HA with 12-fold greater efficiency compared to CS (TABLE 5).

**TABLE 5. The apparent kinetic parameters of PILya44 enzyme.**

| **Substrate** | ***K*ₘ (µg·µl⁻¹)** | ***k*_{cat} (s⁻¹)** | ***k*_{cat}/*Kₘ* (µl·µg⁻¹·s⁻¹)** |
|---|---|---|---|
| HA HMW | 0.6 ± 0.02 | 421 ± 13 | 739 ± 81 |
| CS | 0.8 ± 0.09 | 19 ± 2 | 64 ±6 |

Apparent *k*_{cat} values were calculated from *V*ₘₐₓ values considering a PILya44 theoretic molecular mass of 42107.35 Da. *V*ₘₐₓ refers to the maximum rate of reaction, specifically the micromoles of reducing sugars released per minute.

Chondroitin differs from HA only in the anomeric configuration at the C4 position of the GIcNAc units, which is converted to N-acetyl-D-galactosamine (GaINAc). However, the degradation of CS is influenced by specific sulphation patterns, which further modulate enzymatic activity. While PILya44 demonstrates impressive efficacy in degrading HMW HA, it shows limited activity against other GAGs as CS.

### Production of hyaluronan oligosaccharides using PILya44 hyaluronidase

HPAEC-PAD and MS were employed to analyse the production of HAOS from HMW HA over a 24-hour period using the PILya44 hyaluronidase (FIG 7, TABLE 6, TABLE 7). The reactions were conducted in distilled water using a concentration of 2.5 g/L of HMW HA, with the total reaction volume scaled up to 6 mL. Monitoring the reaction through HPAEC-PAD revealed the formation of HAOS across a diverse range of sizes, with a predominant increase in higher molecular weight oligosaccharides at the initial time points of degradation. This contrasts with the accumulation of smaller HAOS towards the end of the reaction period (FIG 7).

**TABLE 6. Mass spectrometric analysis of the enzymatic degradation of HMW HA using the recombinant PILva44 enzyme. The main ions (in negative mode) and their assignations are shown.**

| | | | | **Intensity (%)** | | | |
|---|---|---|---|---|---|---|---|
| **m/z** | **Observed ion** | **Molecular Mass** | **Oligomer** | **5 min** | **30 min** | **2 h** | **24 h** |
| **380.1** | [M-H₂O+H]⁺ | 379.1 | ΔHA02 | 100.0 | 100.0 | 100.0 | 100.0 |
| **402.1** | [M-H₂O +Na]⁺ | 379.1 | ΔHA02 | 13.0 | 16.3 | 45.9 | 58.8 |
| **420.1** | [M+Na]⁺ | 397.1 | HA02 | - | - | - | 14.5 |
| **578.1** | [M-H₂O+GlcA+Na]⁺ | 555.2 | ΔHA03 | - | 6.4 | 6.6 | 6.6 |
| **583.2** | [M-H₂O+GlcNAc+H]⁺ | 562.2 | ΔHA03 | 14.1 | 15.9 | 19.3 | 25.0 |
| **623.2** | [M+GlcNAc+Na]⁺ | 588.2 | HA03 | - | 2.7 | 3.0 | 14.9 |
| **781.2** | [M₂-H₂O+Na]⁺ | 758.2 | ΔHA04 | 3.0 | 9.2 | 48.6 | 82.0 |
| **799.2** | [M₂+Na]⁺ | 776.3 | HA04 | - | - | 4.0 | 19.0 |
| **957.2** | [M₂-H₂O+GlcA+Na]⁺ | 934.2 | ΔHA05 | - | 3.9 | 4.4 | 5.0 |
| **962.3** | [M₂-H₂O+GlcNAc+Na]⁺ | 939.2 | ΔHA05 | - | - | - | 3.4 |
| **1002.3** | [M₂+GlcNAc+Na]⁺ | 979.2 | HA05 | - | - | - | 9.3 |
| **1160.3** | [M₃-H₂O+Na]⁺ | 1137.3 | ΔHA06 | 2.3 | 5.5 | 21.7 | 20.2 |
| **1178.3** | [M₃+Na]⁺ | 1155.3 | HA06 | - | - | - | 5.8 |
| **1336.3** | [M₃-H₂O+GlcA+Na]⁺ | 1313.3 | ΔHA07 | - | 1.7 | 1.4 | 1.6 |
| **1363.5** | [M₃-H₂O+GlcNAc+Na]⁺ | 1340.3 | ΔHA07 | - | - | - | 0.8 |
| **1381.4** | [M₃+GlcNAc+Na]⁺ | 1358.3 | HA07 | - | - | - | 1.4 |
| **1539.3** | [M₄-H₂O+Na]⁺ | 1516.3 | ΔHA08 | 1.1 | 3.4 | 10.6 | 7.5 |
| **1557.4** | [M₄+Na]⁺ | 1534.3 | HA08 | - | - | - | 2.8 |
| **1715.3** | [M₄-H₂O+GlcA+Na]⁺ | 1692.3 | ΔHA09 | - | 1.4 | - | - |
| **1760.6** | [M₄+GlcNAc+Na]⁺ | 1737.3 | HA09 | - | - | - | 0.8 |
| **1918.4** | [M₅-H₂O+Na]⁺ | 1895.3 | ΔHA10 | 1.4 | 1.8 | 2.8 | 2.3 |
| **1936.5** | [M₅+Na]⁺ | 1913.3 | HA10 | - | - | - | 0.8 |
| **2297.5** | [M₆-H₂O+Na]⁺ | 2274.5 | ΔHA12 | - | 0.9 | 0.7 | - |

The abbreviation 'M' refers to the HA disaccharide, (β-GlcA(1,3)-β-GIcNAc(1 ,4)). Peaks corresponding to [M+H]+ and [M+Na]+ were detected in positive ion mode. The relative intensities (%) indicate the abundance of each ion at different time points: 5 min, 30 min, 2 h, and 24 h. The symbol '-' denotes cases where the ion was not detected.

These large oligomers are progressively broken down into smaller forms over time, leading to the accumulation of smaller oligomers like ΔHA04 at later stages (24 hours), thereby confirming the internal cleavage of the polymer (TABLE 7). In both ionization modes, the accumulation of smaller oligomers such as ΔHA02 in positive ion mode and ΔHA04 in negative ion mode at the 24-hour time point highlights the ability of PILya44 to continue degrading larger oligomers into smaller products. Collectively, these results demonstrate that PILya44 predominantly operates through endo-degradation, initially producing a range of oligomers before progressively breaking them down into smaller fragments throughout the reaction. This contrasts with the mechanism of Streptococcal HA lyases, which proceed via a processive exo-acting mode after an endolytic "initial bite".

**TABLE 7. Mass spectrometric analysis of the enzymatic degradation of HMW HA using the recombinant PILya44 enzyme. The main ions (in positive mode) and their assignations are shown.**

| | | | | **Intensity (%)** | | | |
|---|---|---|---|---|---|---|---|
| **m/z** | **Observed ion** | **Molecular Mass** | **Oligomer** | **5 min** | **30 min** | **2 h** | **24 h** |
| **757.2** | [M₂-H₂O-H]⁻ | 758.2 | ΔHA04 | - | - | 29.2 | 100.0 |
| **933.3** | [M₂-H₂O+GlcA-H]⁻ | 934.2 | ΔHA05 | - | - | 4.3 | 3.9 |
| **1136.3** | [M₃-H₂O-H]⁻ | 1137.3 | ΔHA06 | - | 46.8 | 59.6 | 82.4 |
| **1312.3** | [M₃-H₂O+GlcA-H]⁻ | 1313.3 | ΔHA07 | - | 35.9 | 7.8 | - |
| **1515.4** | [M₄-H₂O-H]⁻ | 1516.3 | ΔHA08 | 100.0 | 100.0 | 100.0 | 8.2 |
| **1691.4** | [M₄-H₂O+GlcA-H]⁻ | 1692.4 | ΔHA09 | - | 46.7 | 4.8 | - |
| **1894.5** | [M₅-H₂O-H]⁻ | 1895.3 | ΔHA10 | 88.3 | 88.0 | 43.5 | - |
| **2070.5** | [M₅-H₂O+GlcA-H]⁻ | 2071.5 | ΔHA11 | - | 19.7 | 1.2 | - |
| **2273.5** | [M₆-H₂O-H]⁻ | 2274.5 | ΔHA12 | 37.7 | 48.8 | 8.5 | - |
| **2449.6** | [M₆-H₂O+GlcA-H]⁻ | 2450.6 | ΔHA13 | - | 8.1 | - | - |
| **2652.7** | [M₇-H₂O-H]⁻ | 2653.7 | ΔHA14 | 13.6 | 17.7 | 1.4 | - |
| **3031.8** | [M₈-H₂O-H]⁻ | 3032.8 | ΔHA16 | 13.7 | 4.4 | 0.3 | - |

The abbreviation 'M' refers to the HA disaccharide, (β-GlcA(1,3)-β-GlcNAc(1,4)). Peaks corresponding to [M-H]+ were detected in positive ion mode. The relative intensities (%) indicate the abundance of each ion at different time points: 5 min, 30 min, 2 h, and 24 h. The symbol '-' denotes cases where the ion was not detected.

Additionally, the observation of odd-numbered unsaturated oligomers of various sizes supports the idea that PILya44 cleaves both β(1,4) bonds between GIcNAc and GlcA and β(1,3) bonds between GlcA and the subsequent GlcNAc residues. Odd-numbered oligosaccharides are generated when β(1,3) glycosidic bonds are cleaved, leaving GlcNAc at the non-reducing end, which is subsequently converted into ΔHexNAc. In the case of PILya44, this dual cleavage of β(1,3) and β(1,4) bonds likely explains the production of such a diverse range of oligosaccharides.

Interestingly, saturated HAOS were also detected (TABLE 6). Although PILya44 primarily generates unsaturated oligosaccharides during polymer cleavage, the terminal fragments that remain at the non-reducing end may persist without significant further degradation. This suggests that these terminal fragments could be saturated, contributing to the overall profile of HAOS produced. Its apparently preference for cleaving longer unsaturated oligomers may also influence the formation of saturated HAOS, generating a diverse range of oligomers, including saturated fragments that are likely to accumulate and be detectable among the reaction products.

These findings highlight PILya44 as a unique, versatile and efficient biocatalyst for HA degradation, with potential applications in industrial, biomedical, and biotechnological settings where controlled generation of hyaluronic acid-derived products is desired (Pang et al., 2022).

### Optimizing large-scale production of PILya44 enzyme and HAOS for evaluating their antioxidant potential

The scale-up of PILya44 production from *P*. *lilacinum,* expressed in *P*. *pastoris,* was performed using the FBMS medium, as described in *Recombinant protein production via fed-batch fermentation* Section of the Materials and Methods. Before inducing protein expression, the selected transformant with optimal PILya44 production capability was cultured in BMG medium. This culture was subsequently used to inoculate the fermenter, achieving an initial OD600 of approximately 0.004. The pH of the fermenter was controlled at 6.0 by the addition of NH₄OH, while agitation was adjusted to maintain dissolved oxygen (pO2) levels above 5 %, with an optimal stabilization around 20 %. Enzymatic activity in the fermenter showed a steady increase (FIG 8), culminating in a 12.6-fold enhancement (7.81 U/mL) compared to activity in the BMM medium with 0.5 % methanol in the flask (0.62 U/mL) (FIG 4).

Peak protein production in the fermenter was reached at 76 hours, while in the flask, it occurred after 120 hours. The fermenter yielded a significantly higher protein concentration, producing 1.02 g/L, 15 times more than the 0.07 g/L achieved in the flask (FIG 9, FIG 5). The yeast extracellular fraction (~2.1 L), processed as outlined in *Recombinant protein production via fed-batch fermentation* Section, was subsequently used for the large-scale production of HAOS.

The degradation of HMW HA was scaled up to 200 mL, with the same reaction conditions as before, except for minor modifications as described in *Large-scale production of HAOS* section of the Materials and Methods. As in earlier experiments, the reaction was performed in distilled water. Two separate reactions were conducted: one terminated after 3 hours, and the other extended to 72 hours, each yielding distinct product profiles (FIG 10, TABLE 8, TABLE 9).

**TABLE 8. Mass spectrometric analysis of the enzymatic degradation of HMW HA using the recombinant PILya44 enzyme obtained from fed-batch fermentation. The main ions (in positive mode) and their assignations are shown.**

| | | | | **Intensity (%)** | |
|---|---|---|---|---|---|
| **m/z** | **Observed ion** | **Molecular Mass** | **Oligomer** | **3 h** | **72 h** |
| **380.1** | [M-H₂O+H]⁺ | 379.1 | ΔHA2 | 100.0 | 6.9 |
| **402.1** | [M-H₂O+Na]⁺ | 379.1 | ΔHA2 | 14.3 | 80.2 |
| **578.1** | [M-H₂O+GlcA+Na]⁺ | 555.2 | ΔHA3 | - | 5.6 |
| **583.2** | [M-H₂O+GlcNAc+H]⁺ | 562.2 | ΔHA3 | 15.4 | - |
| **781.2** | [M₂-H₂O+Na]⁺ | 758.2 | ΔHA4 | 18.3 | 100.0 |
| **957.2** | [M₂-H₂O+GlcA+Na]⁺ | 934.2 | ΔHA5 | - | 0.9 |
| **1160.3** | [M₃-H₂O+Na]⁺ | 1137.3 | ΔHA6 | 8.2 | 7.2 |
| **1539.3** | [M₄-H₂O+Na]⁺ | 1516.3 | ΔHA8 | 3.9 | 0.2 |
| **1918.4** | [M₅-H₂O+Na]⁺ | 1895.3 | ΔHA10 | 0.8 | - |

The abbreviation 'M' refers to the hyaluronic acid disaccharide, (β-GlcA(1,3)-β-GlcNAc(1,4)). Peaks corresponding to [M+H]+ and [M+Na]+ were detected in positive ion mode. The relative intensities (%) indicate the abundance of each ion at different time points: 3 h and 72 h. The symbol '-' denotes cases where the ion was not detected.

**TABLE 9. Mass spectrometric analysis of the enzymatic degradation of HMW HA using the recombinant PILya44 enzyme obtained from fed-batch fermentation. The main ions (in negative mode) and their assignations are shown.**

| **Intensity (%)** | | | | | |
|---|---|---|---|---|---|
| **m/z** | **Observed ion** | **Molecular Mass** | **Oligomer** | **3 h** | **72 h** |
| **757.2** | [M₂-H₂O-H]⁻ | 758.2 | ΔHA4 | 17.2 | 100.0 |
| **1136.3** | [M₃-H₂O-H]⁻ | 1137.3 | ΔHA6 | 52.9 | 40.4 |
| **1515.4** | [M₄-H₂O-H]⁻ | 1516.3 | ΔHA8 | 100.0 | - |
| **1553.4** | [M₄-H₂O-2H+K]⁻ | 1516.3 | ΔHA8 | 4.1 | 1.6 |
| **1894.5** | [M₅-H₂O-H^{]-} | 1895.3 | ΔHA10 | 39.7 | - |
| **2273.5** | [M₆-H₂O-H]⁻ | 2274.5 | ΔHA12 | 9.3 | - |
| **2652.7** | [M₇-H₂O-H]⁻ | 2653.7 | ΔHA14 | 2.6 | - |

The abbreviation 'M' refers to the hyaluronic acid disaccharide, (β-GlcA(1,3)-β-GlcNAc(1,4)). Peaks corresponding to [M-H]- were detected in negative ion mode. The relative intensities (%) indicate the abundance of each ion at different time points: 3 h and 72 h. The symbol '-' denotes cases where the ion was not detected.

As described in *Large-scale production of HAOS* section, product mixtures purification was carried out using VivaFlow membranes with a 30 kDa cutoff to remove the enzyme. After freeze-drying, the products were characterized via HPAEC-PAD (FIG 10) and MS (TABLE 8, TABLE 9). The 3-hour reaction yielded approximately 44 % HAOS, corresponding to 0.22 g of product from 0.5 g of HMW HA. The 72-hour reaction yielded about 90 %, or 0.45 g of product from 0.5 g of HMW HA. The product profiles of both reactions were consistent with previously obtained results. PILya44 primarily generated even-numbered unsaturated HAOS, although odd-numbered unsaturated oligomers were also detected in both product mixtures (TABLE 8, TABLE 9). No saturated oligosaccharides were observed, highlighting the complexity of the reaction kinetics and HA degrading mechanism.

The antioxidant activity of the HAOS mixtures obtained was evaluated using the ABTS·+ radical discoloration assay, with Trolox serving as the reference antioxidant. The half-maximal scavenging concentration (SC₅₀) was defined as the concentration (mg/mL) required to reduce the absorbance of the ABTS·+ radical by 50 % within 15 min. The antioxidant activity of HMW HA and the obtained product mixtures, along with commercial HAOS products (HyaloOligos, HyaloBooster; see TABLE 4 above), was assessed across final concentrations ranging from 0 to 0.4 mg/mL for HMW HA, and from 0 to 12 mg/mL for HyaloOligos, HyaloBooster, and the two obtained mixtures (HAOS-3h and HAOS-72h), as detailed in *Antioxidant Activity* Section. Previous studies demonstrated strong, dose-dependent radical scavenging properties of unsaturated HAOS derived from heterologous expressed HA lyase from *S*. *pyogenes* bacteriophage H4489A using the DPPH free radical assay (El-Safory, N. S., & Lee, C. K. (2010). Cytotoxic and antioxidant effects of unsaturated hyaluronic acid oligomers. Carbohydrate Polymers, 82(4), 1116-1123. https://doi.org/10.1016/j.carbpol.2010.06.042).

The SC₅₀ values were calculated for all compounds tested, along with the TEAC values (see TABLE 10). Due to the non-normal distribution of the data, the Kruskal-Wallis test was employed, revealing significant differences in SC₅₀ values (Kruskal-Wallis statistic = 16.51, p = 0.0055). Dunn's multiple comparisons test further identified that Trolox exhibited a significantly lower SC₅₀ value compared to HMW HA (p = 0.0086), indicating that HMW HA had no effective antioxidant activity within the tested concentration range.

The results indicate that the HAOS mixtures possess notable antioxidant properties, with the HAOS-72h mixture showing the most promising activity among the tested mixtures (TABLE 10), indicating that prolonged reaction times enhance antioxidant potential. Interestingly, HyaloBooster, the HAOS-3h and HAOS-72h mixtures, consisting predominantly of unsaturated oligosaccharides, displayed antioxidant potential exceeding that of HyaloOligos.

**TABLE 10. Half-maximal scavenging activity (SC₅₀) of high molecular weight hyaluronate and derivatives compared with the reference antioxidant Trolox.**

| **Compound** | **SC₅₀ (mg/mL)** |
|---|---|
| **HMW HA** | 36 ± 3 * |
| **HyaloOligos** | 4.9 ± 0.5 |
| **HyaloBooster** | 0.9 ± 0.01 |
| **HAOS-3h** | 0.9 ± 0.01 |
| **HAOS-72h** | 0.5 ± 3·10⁻³ |
| **Trolox** | 0.004 ± 8·10⁻⁵ * |

SC₅₀ refers to the concentration required to achieve 50 % scavenging activity. The TEAC values were calculated based on the obtained SC50 values. * Statistically significant difference (p < 0.01) between HMW HA and Trolox.

A correlation between molecular weight and radical scavenging activity was also observed. Polysaccharides with lower molecular weights typically possess more accessible reductive hydroxyl groups, facilitating free radical elimination. As polymers degrade, the reduction in molecular weight enhances the antioxidant potential of polysaccharides, which has been previously report for LMW HA compared to HMW HA. Consequently, the degradation of HMW HA into oligosaccharides significantly contributes to the improved antioxidant activity of HAOS. The presence of glucuronic acid sugar units, with electron-donating groups may explain the observed antioxidant activities. This has been similarly observed in the case of unsaturated pectin oligosaccharides formed through enzymatic depolymerization and with xylo-oligosaccharides from beechwood xylan.

These findings highlight the importance of molecular weight and unsaturations in HAOS for their antioxidant properties and confirm the potential of the HAOS mixtures produced in this study for applications requiring enhanced antioxidant capacity.

## Claims

1. A polypeptide with hyaluronidase activity comprising:
the amino acid sequence in SEQ ID No. 1 or
a variant thereof, with an amino acid sequence having at least 80% at least 85%, at least 90% at least 95%, at least 96%, at least 97%, at least 98%, at least 99%, sequence identity over the whole length of SEQ ID No. 1, the variants having hyaluronidase activity.

2. The polypeptide with hyaluronidase activity from the preceding claim, being an isolated polypeptide and/or a recombinant polypeptide

3. The polypeptide with hyaluronidase activity from any of the preceding claims, obtained from *Purpureocillium sp,* preferably *Purpureocillium sp* is selected from the group consisting of P. *lilacinum*, *P. lavendulum* and *P*. *takamizusanensis.*

4. The polypeptide with hyaluronidase activity from any of the preceding claims, comprising a signaling peptide, preferably SEQ ID No. 3.

5. The polypeptide with hyaluronidase activity from any of the preceding claims, wherein the variants have at least 20%, or, at least 40%, at least 50%, at least 60%, at least 70%, at least 80%, at least 90%, at least 95%, or at least 100% of the hyaluronidase activity of SEQ ID NO: 1.

6. A polynucleotide comprising the nucleotide sequence shown in SEQ. ID. No. 5 encoding the hyaluronidase of any of the preceding claims 1 to 4, or a variant thereof having at least 80% at least 85%, at least 90% at least 95%, at least 96%, at least 97%, at least 98%, at least 99%, sequence identity over the whole length of SEQ ID No. 5.

7. The polynucleotide according to the preceding claim wherein the variant is a codon-optimized variant for a host cell, preferably, the host cell is *P. pastoris* and preferably the variant is SEQ. ID. No. 9

8. A genetically engineered recombinant vector comprising a polynucleotide according to any of the preceding claims 6 or 7.

9. A vector according to claim 8, wherein the vector is a plasmid, preferably a pGem-T plasmid or a plB4 plasmid.

10. A host cell comprising the vector according to any of the preceding claims 8 or 9.

11. The host cell according to the preceding claim, being a prokaryote or a eukaryote host cell, preferably *P*. *pastoris.*

12. A method to produce the polypeptide with hyaluronidase of SEQ ID No 1 or a variant thereof as described in any of the preceding claims 1 to 5, from a host cell comprising the polynucleotide SEQ ID No 5 or a variant thereof as described in the preceding claim, comprising the steps of:
a. cultivating a host cell of claim 11 under conditions suitable for expression of the polypeptide; and
b. recovering a polypeptide of step a.

13. The method according to the preceding claim, wherein the conditions suitable for expression are temperature from 20°C to 40°C; agitation from 300 rpm to 2000 rpm; dissolved O₂ from 10% (v/v) to 30% (v/v); and pH, between 3.5 and 8.

14. Use of SEQ ID No 1 or a variant thereof to produce a mixture of hyaluronan oligosaccharides comprising un-saturated, odd, and even numbered HAOS; comprising the steps of:
a. putting in contact the SEQ ID No 1 or a variant thereof with: HMW-HA, MMW-HA, LMW-HA or mixtures thereof, in a suitable medium,
b. let the reaction happen for a suitable time, such as 5 min and 200 h.

15. The use according to the preceding claim wherein the temperature of the reaction is between 39°C and 55°C, and the pH of the reaction between 3.5 and 6.
